# FASCICULE DE BREVET EUROPEEN

(11) **EP 1 652 553 B1**
(45) Date de publication et mention de la délivrance du brevet: **03.06.2009**
(21) Numéro de dépôt: 05291878.6
(22) Date de dépôt: 12.09.2005
(51) Int. Cl.: A61Q 5/06, A61K 8/49

(54) **Composition comprenant au moins un dérivé substitué de carbocyanine, procédé de traitement des fibres kératiniques la mettant en oeuvre, dispositif et utilisation**
Zusammensetzung enthaltend zumindest ein Carbocyanin, Behandlungsprozess für keratisnische Fasern, Kit und Verwendung dieser Zusammensetzung
Composition comprising at least one carbocyanine, process of treating keratinic fibres, kit and use of said composition

(30) Priorité: 13.09.2004 FR 0409694
(43) Date de publication de la demande: 03.05.2006
(73) Titulaire: L'ORÉAL, 75008 Paris (FR)
(72) Inventeur: Lagrange, Alain, 77700 Coupvray (FR)
(74) Mandataire: Fevrier, Murielle Françoise E.

(56) Documents cités:
- WO-A-99/13849
- WO-A-99/31181
- US-A- 3 679 427
- US-A- 3 864 644

## Description

L'invention concerne une composition comprenant, dans un milieu cosmétiquement acceptable, au moins un colorant direct particulier. Elle a de même pour objet un procédé de traitement de fibres kératiniques mettant eh jeu cette composition, ainsi qu'un dispositif la comprenant. Enfin, elle a pour objet l'utilisation de la composition selon l'invention comme agent colorant desdites fibres.

La présente invention a trait au domaine de la coloration des fibres kératiniques et plus particulièrement de la coloration capillaire.

Il existe essentiellement deux types de coloration.

Le premier est la coloration dite semi-permanente ou coloration directe, qui fait appel à des colorants capables d'apporter à la coloration naturelle des cheveux, une modification plus ou moins marquée.
Les colorants mis en jeu sont des substances colorées et colorantes qui présentent une certaine affinité avec la fibre kératinique.
Il est à noter que ce type de coloration s'estompe au bout de plusieurs lavages, ce qui peut représenter un inconvénient.

Dans le cas où l'on souhaite obtenir une coloration plus claire que la couleur originale des fibres, il est nécessaire d'utiliser avec les colorants directs, au moins un agent oxydant, dans des conditions de pH alcalin.
Cependant, ces conditions de mise en oeuvre ne sont pas sans conséquence sur les propriétés des fibres traitées. En effet, à la longue, les fibres sont plus ou moins dégradées et ont tendance à devenir rêches, ternes, cassantes, difficiles à coiffer.

Le deuxième est la coloration permanente ou coloration d'oxydation. Celle-ci est réalisée avec des précurseurs de colorants d'oxydation qui sont des composés incolores ou faiblement colorés, comprenant au moins une base d'oxydation éventuellement associée à un ou plusieurs coupleurs. Une fois mélangés à des produits oxydants, au moment de l'emploi, les précurseurs donnent naissance par un processus de condensation oxydative à des composés colorés et colorants.

Etant donné la présence nécessaire d'un agent oxydant dans ce type de coloration, les inconvénients mentionnés ci-dessus se retrouvent aussi dans ce cas.

Par ailleurs, Il a été montré que les colorants fluorescents peuvent présenter un intérêt en coloration capillaire.

Cependant la stabilité en conservation des compositions contenant des colorants fluorescents classiques peut être améliorée.
Par ailleurs, il serait aussi avantageux d'augmenter encore la ténacité aux lavages et aux shampooings des colorations obtenues au moyen de ces compositions.

Il a été trouvé de manière totalement inattendue que des compositions comprenant au moins un composé direct correspondant à un dérivé substitué de carbocyanine particulier permettait d'obtenir des résultats de coloration encore plus satisfaisants avec un bonne stabilité des compositions. Par ailleurs, la plupart des composés mis en oeuvre dans le carde de la présente invention présentent l'avantage d'être fluorescents, leur utilisation permet également de résoudre le problème évoqué ci-dessus.

La présente invention a donc pour premier objet une composition de coloration de fibres kératiniques, notamment humaines, comprenant, dans un milieu cosmétiquement acceptable comprenant de l'eau ou un mélange d'eau et de solvant organique, au moins un agent tensioactif et/ou au moins un polymère épaississant, et, au moins un colorant direct soluble dans le milieu, de formules (I) ou (I') suivantes : 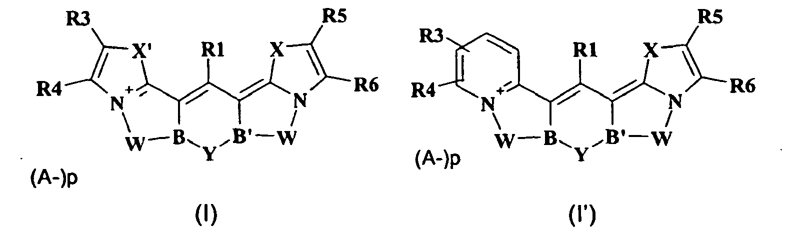 dans lesquelles :
R1 représente un atome d'hydrogène, un radical alkyle linéaire, ramifié, cyclique ou comprenant 1 à 22 atomes de carbone, plus particulièrement 1 à 6 atomes de carbone, éventuellement substitué par au moins un groupement hydroxyle, par au moins un groupement alcoxy en C₁-C₆ linéaire ou ramifié, par au moins un groupement cycloalcoxy en C₁-C₆, par au moins un groupement phényle lui-même éventuellement substitué par au moins un groupement carboxy, un radical aryle en C₆-C₃₀, un radical amino substitué par un ou plusieurs radicaux identiques ou non, alkyle ou hydroxyalkyle en C₁-C₆, linéaires ou ramifiés R₃, R₄, R₅ et R₆ indépendamment l'un de l'autre, représentent un atome d'hydrogène ; un radical alkyle, linéaire ou ramifié, comprenant 1 à 22 atomes de carbone, plus particulièrement 1 à 10 atomes, de préférence de 1 à 6 atomes de carbone, éventuellement substitué par un ou plusieurs radicaux hydroxyle ; un atome d'halogène, un radical carboxy, un radical sulfo ;
R₃ et R₄ et/ou R₅ et R₆ peuvent former deux à deux avec les atomes de carbone auquel chacun est rattaché, un cycle ou un hétérocycle aliphatique ou aromatique comprenant de 6 à 30 chaînons, éventuellement condensé à un cycle ou hétérocycle aromatique identique ou non comprenant de 6 à 30 chaînons ; les cycles ou hétérocycles aliphatiques ou aromatiques étant éventuellement substitués par au moins un atome d'halogène ; par au moins un radical alcoxy en C₁-C₆, par au moins un radical carboxy, par au moins un radical sulfo et/ou par au moins un radical aryle en C₆-C₃₀ par au moins un radical alkyle linéaire ou ramifié en C₁-C₆, ledit radical alkyle étant éventuellement interrompu par un groupement aminocarbonyle ou carbonylamino et éventuellement terminé par un groupement hydroxyle, carboxy, amino, hydrogénocarbonylamino.
B, B' indépendamment l'un de l'autre représentent un atome d'azote ou un groupement CH ;
W représente un radical divalent comportant deux atomes de carbone, de telle sorte que l'enchaînement N-W-B comporte ou non une insaturation, ledit radical divalent étant éventuellement substitué par une chaîne alkyle en C₁-C₆, aryl(C₆-C₃₀)oxy alkyle(C₁-C₆), ou alkyl(C₁-C₄)arylC₆-C₃₀)amino ;
X, X' indépendamment l'un de l'autre représentent O, S, N, NR'₇, Se, CR'₈R'₉ ;
Y représente O, S, N, Se, NR₇, CO, CR₈R₉ ;
R₈, R₉ indépendamment l'un de l'autre, représentent un atome d'hydrogène ; un radical alkyle linéaire ou ramifié en C₁-C₂₂ éventuellement substitué par un ou plusieurs radicaux hydroxyle, mono ou dialkylamino en C₁-C₁₀, mono ou dihydroxylalkylamino en C₁-C₁₀, aryle en C₁₀-C₃₀, aryloxy en C₁₀-C₃₀, acyl(C₂-C₁₀)amino ;
R₇ représente un atome d'hydrogène, un radical alkyle linéaire ou ramifié en C1-C10, un radical aryle en C6-C30, un radical amino porteur d'au moins un radical aryle en C₆-C₃₀ éventuellement substitué par au moins un radical alkyle en C₁-C₆ ou au moins un radical carboxy, d'au moins un radical alkyl(C₁-C₄)aryl(C₆-C₃₀)sulfonyle, ou au moins un radical acyle en C₂-C₁₀ ou au moins un radical trialkyl(C₁-C₄)aminoalkyl(C₁-C₄)carbonyle, ou au moins un groupement aminothiocarbonyle ;
R₇ peut aussi représenter un radical Z ou -N=Z, Z désignant un hétérocycle à 5 ou 6 chaînons éventuellement condensé et comportant de 1 à 30 atomes de carbone, un ou plusieurs des atomes de carbone pouvant être remplacé par un groupement CO ;
R'₇ représente un atome d'hydrogène, un radical alkyle en C₁-C₆, un radical hydroxyalkyle en C₁-C₆ ;
R'₈ et R'₉ indépendamment l'un de l'autre représentent un atome d'hydrogène, un radical alkyle en C₁-C₆, un radical hydroxyalkyle en C₁-C₆, un radical carboxyalkyle en C₁-C₆, un radical alcoxyC₁-C₄carbonylyalkyle en C₁-C₆;
p est un entier égal à 0 ou 1 ;
A- représente un anion organique ou minéral, ou un mélange d'anions.

Elle a de même pour objet un procédé de traitement de fibres kératiniques plus particulièrement de fibres kératiniques humaines, dans lequel applique sur lesdites fibres, sèches ou humides, la composition selon l'invention, pendant un temps suffisant pour développer la coloration, après quoi on rince, on lave éventuellement au shampooing, on rince à nouveau et on sèche ou on laisse sécher les fibres résultantes.

Selon une variante du procédé, on applique sur lesdites fibres, sèches ou humides, la composition selon l'invention, sans rinçage final.

Un autre objet de l'invention est constitué par un dispositif à plusieurs compartiments dont au moins l'un comprend la composition selon l'invention et au moins un autre comprend un agent oxydant.

Enfin, l'invention concerne l'utilisation de la composition selon l'invention comme agent colorant des fibres kératiniques, en particulier humaines.

Mais d'autres caractéristiques et avantages de l'invention apparaîtront plus clairement à la lecture de la description et des exemples qui vont suivre.

Dans ce qui va suivre et à moins qu'une indication différente ne soit indiquée, les bornes d'un domaine de valeurs sont comprises comme faisant partie de ce domaine.

Selon la présente invention, on entend par fibres kératiniques humaines, les cheveux, les cils, et les sourcils.
Il est à noter que la composition est appropriée pour le traitement de fibres kératiniques, notamment humaines, quelle que soit leur coloration avant traitement et que celle-ci soit naturelle ou obtenue artificiellement.

Selon un mode de réalisation avantageux de l'invention, la composition est destinée à être appliquée sur des fibres kératiniques, notamment humaines, foncées. Plus particulièrement, les fibres kératiniques foncées sont des fibres pigmentées ou colorées artificiellement, dont la hauteur de ton est inférieure ou égale à 6 et de préférence inférieure ou égale à 4.

Font également partie du cadre de la présente invention, les formes mésomères des composés de formule (I) ou (I').

Certains des composés de formule (I) ou (I') sont fluorescents.
Un colorant fluorescent est plus spécialement une molécule qui colore par elle-même, est soluble dans le milieu, absorbe la lumière du spectre visible et en outre éventuellement de l'ultraviolet (longueurs d'onde allant de 360 à 760 nanomètres) mais qui, contrairement à un colorant classique, transforme une partie de l'énergie absorbée en lumière fluorescente de plus grande longueur d'onde que celle absorbée, émise dans la partie visible du spectre.

Selon un mode de réalisation plus particulier de l'invention, le radical R₁ est un atome d'hydrogène, un radical alkyle linéaire ou ramifié en C₁-C₁₆ non substitué.

Par ailleurs, Y représente de préférence O, N, NR₇, CO.

Selon une variante de l'invention, X et X' désignent indépendamment l'un de l'autre un atome de soufre, un atome d'oxygène, un groupement NR'₇ avec R représentant un radical alkyle en C₁-C₄.
De préférence, X et X' sont identiques.

Conformément à un mode préféré de l'invention, W désigne un radical éthylène.

Selon une variante de l'invention, B et B' désignent un atome de carbone ou un groupement CH.

Conformément à une autre variante de l'invention, R₃ et R₄ et/ou R5 et R6 forment deux à deux avec les atomes de carbone auquel chacun est rattaché, un cycle ou un hétérocycle aliphatique ou aromatique comprenant de 6 à 30 chaînons condensé à un cycle ou hétérocycle aromatique identique ou non comprenant de 6 à 30 chaînons ; les cycles ou hétérocycles aliphatiques ou aromatiques étant éventuellement substitués par au moins un atome d'halogène ; par au moins un radical alcoxy en C₁-C₆, par au moins un radical carboxy, par au moins un radical sulfo et/ou par au moins un radical aryle en C₆-C₃₀ par au moins un radical alkyle linéaire ou ramifié en C₁-C₆, ledit radical alkyle étant éventuellement interrompu par un groupement aminocarbonyle ou carbonylamino et éventuellement terminé par un groupement hydroxyle, carboxy, amino, hydrogénocarbonylamino.

Il est à noter que A⁻ peut être un anion d'origine minérale choisi notamment parmi les halogénures, les sulfates, les bisulfates, les nitrates, les phosphates, les hydrogénophosphates, les dihydrogénophosphates, les carbonates, les bicarbonates.

L'anion A⁻ peut aussi être d'origine organique, et dans ce cas, plus particulièrement choisi parmi ceux provenant de sels d'acides mono- ou polycarboxyliques, sulfoniques, sulfuriques, saturés ou non, aromatiques ou non, éventuellement substitués par au moins un radical hydroxyle, amino, ou atomes d'halogène.

De préférence, A⁻ est choisi parmi le chlorure, l'iodure, le sulfate, le méthosulfate, l'éthosulfate.

De préférence, le ou les composés de formule (I) ou (I') utilisés sont solubles dans le milieu cosmétiquement acceptable.
Plus particulièrement, le colorant direct est soluble dans le milieu de la composition à au moins 1 gramme par litre et de préférence à au moins 5 grammes par litre à la température de 25°C.

Conformément à un mode de réalisation particulièrement avantageux de l'invention, le composé direct correspond à l'une des formules suivantes :

| | |
|---|---|
| Iodure de Benzimidazo[2,1-f] benzimidazo[1',2':1,2]pyrido[4,3- b][1,6]naphthyridinium, 2,3,13,14-tetrachloro-16,18-diethyl-6, 7, 7a,8,8a,9,10,16-octahydro-8-[(4-methylphenyl)amino] | 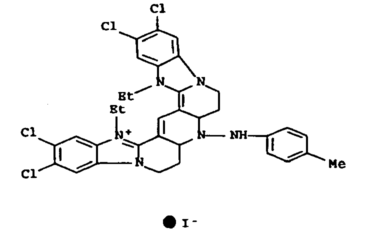 |
| Iodure de 6H-Benzothiazolo[2,3-f]benzothiazolo[3',2':1,2]pyrido[4,3-b][1,6]naphthyridin-5-ium, 8-(2-benzothiazolylamino)-7,7a,8,8a,9,10-hexahydro | 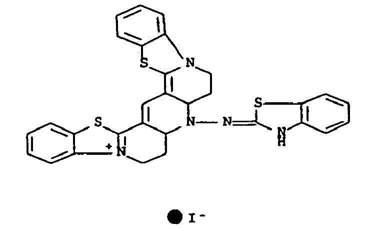 |
| Chlorure iodure de 6H-Benzothiazolo[2,3-f]benzothiazolo[3',2':1,2]pyrido[4,3-b][1,6]naphthyridin-5-ium, 7,7a,8,8a,9,10-hexahydro-8-[[(trimethylammonio) acetyl]amino]-, | 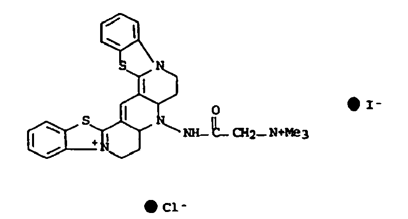 |
| Iodure de 6H-Benzothiazolo[2,3-f]benzothiazolo[3',2':1,2]pyrido[4,3-b][1,6]naphthyridin-5-ium, 7,7a,8,8a,9,10-hexahydro-8-(4-oxo-2-thioxo-3-thiazolidinyl) | 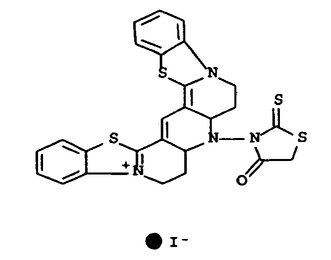 |
| Iodure de 6H-Benzothiazolo[2,3-f] benzothiazolo[3',2':1,2]pyrido[4,3-b][1,6]naphthyridin-5-ium, 7,7a,8,8a,9,10-hexahydro-8-[[(4-methylphenyl)sulfonyl]amino] | 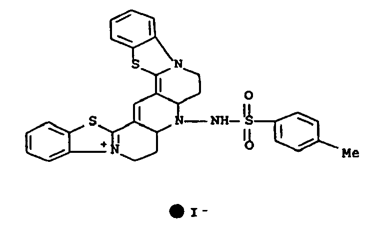 |
| Iodure de 6H-Benzothiazolo[2,3-f]benzothiazolo[3',2':1,2]pyrido[4,3-b][1,6]naphthyridin-5-ium, 8-[(1,1-dioxido-1,2-benzisothiazol-3-yl)amino]-7,7a,8,8a,9,10-hexahydro | 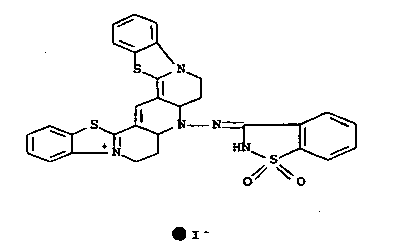 |
| Trifluoroacétate de6H-Pyrano[3",2":3,4;5",6":3',4']dipyrido[1,2-a:1',2'-a']bisbenzimidazolium, 2,3,13,14-tetrachloro-16,18-diethyl-7,7a,8a,9,10,16-hexahydro | 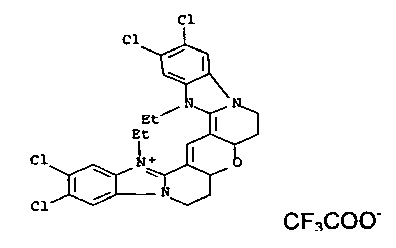 |
| Bromure de 6H,10H-Pyrano[3",2":3,4;5",6":3',4']dipyrido[2,1-b:2',1'-b']bisbenzoxazol-5-ium, 17-ethyl-7,7a,8a,9-tetrahydro-3,13-diphenyl | 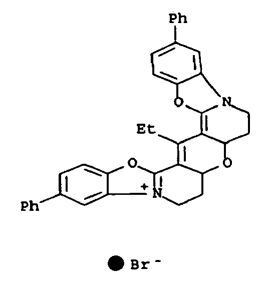 |
| Iodure de 6H,10H-Pyrano [3", 2":3,4; 5",6":3',4']dipyrido[2,1-b:2',1'-b']bisbenzothiazol-5-ium, 7,7a,8a,9-tetrahydro-3,13-dimethoxy | 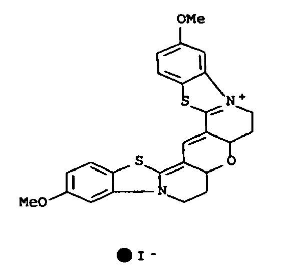 |
| Bromure de 6H,10H-Pyrano [3",2":3,4;5",6":3',4']dipyrido[2,1-b:2',1'-b']bisbenzothiazol-5-ium, 17-ethyl-7,7a,8a,9-tetrahydro | 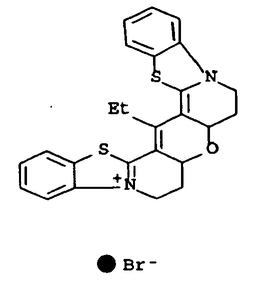 |
| Iodure de 6H,10H-Pyrano[3",2":3,4;5",6":3',4']dipyrido[2,1-b:2',1'-b']bisbenzothiazol-5-ium, 7,7a,8a,9-tetrahydro | 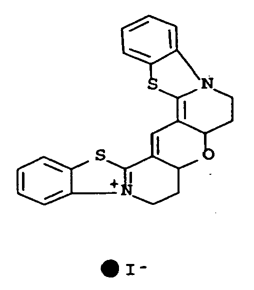 |
| Bromure de 6H-Benzoxazolo[2,3-f]benzoxazolo[3',2':1,2]pyrido[4,3-b][1,6]naphthyridin-5-ium, 8-(2-benzothiazolylamino)-7,7a,8,8a,9,10-hexahydro-3,13-diphenyl | 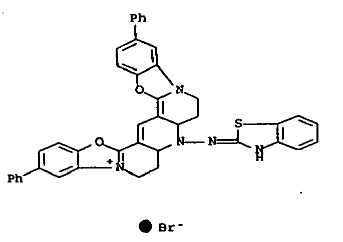 |
| Bromure de 6H-Benzothiazolo[2,3-f]benzothiazolo[3',2': 1,2]pyrido[4,3-b][1,6]naphthyridin-5-ium, 8-(2-benzothiazolylamino)-7,7a,8,8a,9,10-hexahydro | 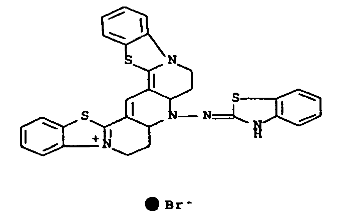 |
| Iodure de 6H-Benzothiazolo[2,3-f]benzothiazolo[3',2': 1,2]pyrido[4,3-b][1,6]naphthyridin-5-ium, 8-[(aminothioxomethyl)amino]-7,7a,8,8a,9,10-hexahydro | 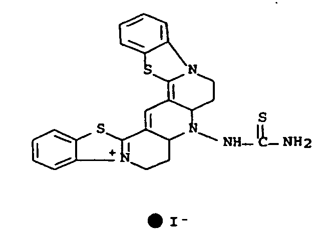 |
| Iodure de 6H-Benzimidazo[2,1-f]benzimidazo[1',2':1,2]pyrido[4,3-b][1,6]naphthyridin-5-ium, 2,3,13,14-tetrachloro-16,18-diethyl-7,7a,8,8a,9,10,16,18-octahydro-9-[(4-methylphenyl)amino] | 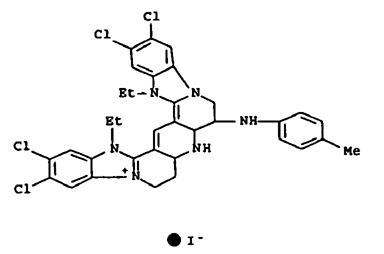 |
| Chlorure de 9H-Bisbenzimidazo [2',1':3,4]pyrazino[1,2-c:2',1'-f]pyrimidin-5-ium, 6,7,11,12,18,20-hexahydro-9-oxo- | 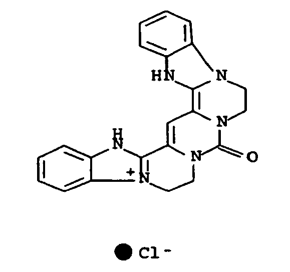 |
| Sel interne de Pyrano [3",2":3,4;5",6":3',4']dipyrido[1,2-a:1',2'-a']diindol-5-ium, 2-(aminomethyl)-6,7,9,10,16,18-hexahydro-16,16,18,18-tetramethyl-14-sulfo- | 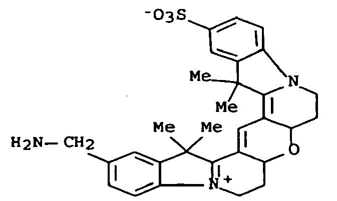 |
| Sel interne de Pyrano [3",2":3,4;5",6":3',4']dipyrido[1,2-a:1',2'-a']diindol-5-ium, 2-(carboxymethyl)-6,7,9,10,16,18-hexahydro-16,16,18,18-tetramethyl-14-sulfo | 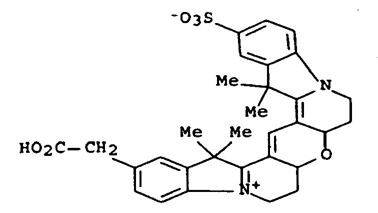 |
| Sel interne de Pyrano [3",2":3,4;5",6":3',4']dipyrido[1,2-a:1',2'-a']diindol-5-ium, 2,14-bis(carboxymethyl)-6,7,9,10,16,18-hexahydro-16,16,18,18-tetramethyl | 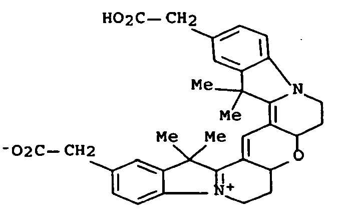 |
| Sel interne de 6H-Benz[2",3"] indolizino[8',7':5,6]pyrano[3,2-a]benzo[f]quinolizin-5-ium, 14-(carboxyméthyl)-7,7a,8a,9,10,16-hexahydro-16,16-dimethyl | 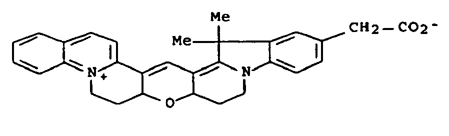 |
| Sel interne de 6H-Benz[2,3]indolizino[7,8-b]indolo[2,1-f][1,6]naphthyridin-5-ium, 8-[(4-carboxyphényl)amino]-7,7a,8,8a,9,10,16,18-octahydro-16,16,18,18-tetramethyl | 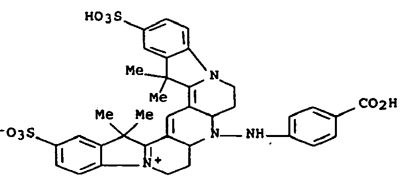 |
| Sel interne de 10H-Benz[2",3"] indolizino[8",7":5',6']pyrano[3',2':3,4]pyrido[ 2,1-b]benzothiazol-5-ium, 2-(carboxymethyl)-6,7,7a,8a,9,18-hexahydro-18,18-dimethyl | 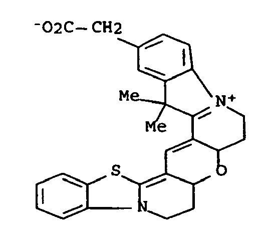 |
| Sel interne de Pyrano [3",2":3,4;5",6":3',4']dipyrido[1,2-a:1',2'-a']diindol-5-ium, 6,7,9,10,16,18-hexahydro-2-(2-hydroxyethyl)-16,16,18,18-tetramethyl-14-sulfo | 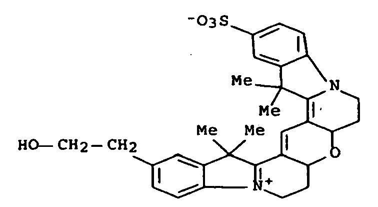 |
| Sel interne de Pyrano [3",2":3,4;5",6":3',4']dipyrido[1,2-a:1',2'-a']diindol-5-ium, 2-[(formylamino)methyl]-6,7,9,10,16,18-hexahydro-16,16,18,18-tetramethyl-14-sulfo | 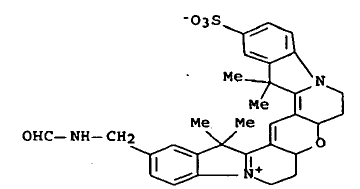 |
| Sel interne de Pyrano [3",2":3,4;5",6":3',4']dipyrido[1,2-a:1',2'-a']diindol-5-ium, 2-[2-[(2-aminoethyl)amino]-2-oxoethyl]-6,7,9,10,16,18-hexahydro-16,16,18,18-tetramethyl-14-sulfo | 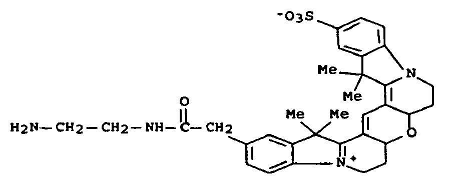 |
| Sel interne de Pyrano [3",2":3,4;5",6":3',4']dipyrido[1,2-a:1',2'-a']diindol-5-ium, 2-[2-[(2-carboxymethyl)amino]-2-oxoethyl]-6,7,9,10,16,18-hexahydro-,16,16,18,18-tetramethyl-14-sulfo | 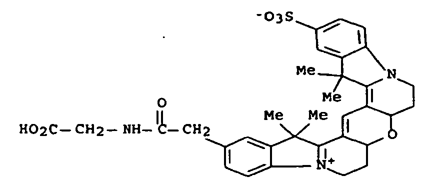 |
| Sel interne de Dibenzo[e,e']pyrano [3",2":3,4;5",6":3',4']dipyrido[1,2-a:1',2'-a']diindol-7-ium, 8,9,11,12,20,22-hexahydro-20,20,22,22-tetramethyl-3,17-disulfo | 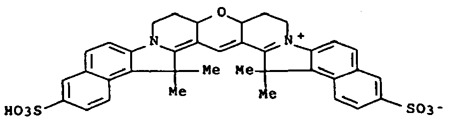 |
| Sel (par exemple chlorure) de 7H,11 H-Bisnaphth[2',3':4,5]oxazolo[3,2-a:3',2'-a']pyrano[3,2-c:5,6-c']dipyridin-6-ium | 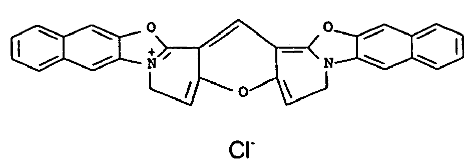 |
| Sel (par exemple chlorure) de 4-methylbenzenesulfonate de 7H,11H-Bisnaphth[2',3':4,5]oxazolo[3,2-a:3',2'-a'] pyrano [3,2-c:5,6-c']dipyridin-6-ium, 20-ethyl-8,8a,9a,10-tetrahydro-8,10-bis(3-phenoxypropyl)-, | 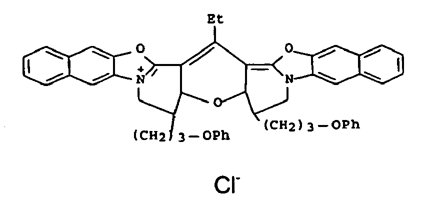 |
| Sel (par exemple chlorure) de 7H,11H-Bisnaphth[2',3':4,5]oxazolo[3,2-a:3',2'-a']pyrano[3,2-c:5,6-c']dipyridin-6-ium, 8,8a,9a,10-tetrahydro-8,10-bis(3-phenoxypropyl)- | 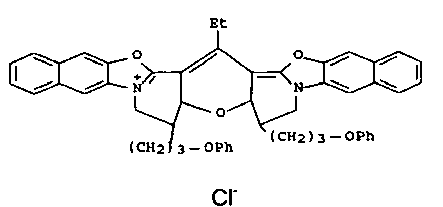 |
| Sel (par exemple chlorure) de 6H,10H-Naphtho[1 "",2"":4"',5"']thiazolo[3"',2"':1",2"] pyrido[3",4":5',6']pyrano[3',2':3,4 ]pyrido[2,1-b]benzoxazol-5- | 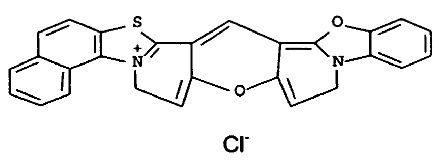 |
| Bromure de 6H,10H-Pyrano[3",2":3,4;5",6":3',4']dipyrido[2,1-b:2',1'-b']bisbenzothiazol-5-ium, 7,7a,8a,9-tetrahydro | 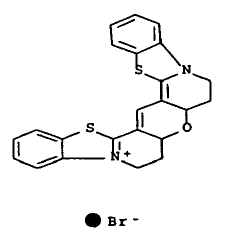 |
| Sel (par exemple chlorure) de 10H-Benz[2",3"]indolizino[8",7":5',6] pyrano[3',2':3,4]pyrido[2,1-b]benzothiazol-5-ium | 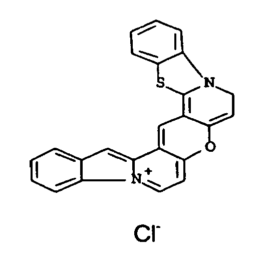 |
| Sel (par exemple chlorure) de Dibenzo[e,e']pyrano[3",2":3,4;5",6":3',4']dip yrido[1,2-a:1',2'-a']diindol-7- | 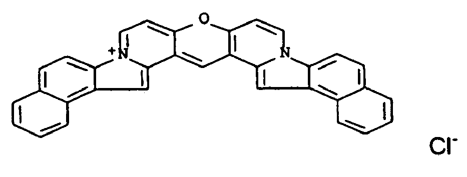 |
| Sel (par exemple chlorure) de Pyrano [3",2":3,4;5",6":3',4']dipyrido[1,2-a:1',2'-a']diindol-5-ium | 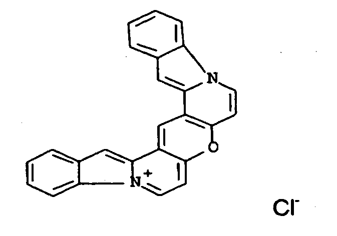 |
| Sel (par exemple chlorure) de 1 H-Benz[2,3]indolizino[7,8-b]indolo[2,1-f][1,6]naphthyridin-5-ium | 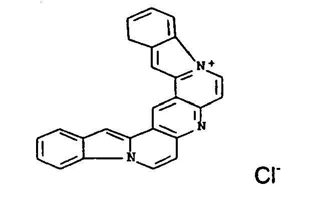 |
| Heptafluorobutanoate 6H,10H-Pyrano[3",2":3,4;5",6":3',4']dipyrido[2,1 -b:2',1'-b']bisbenzothiazol-5-ium, 7,7a,8a,9-tetrahydro-, | 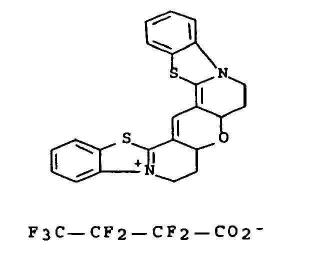 |
| Sulfate de 6H-Pyrano [3",2":3,4;5",6":3',4']dipyrido[1,2-a:1',2'-a']bisbenzimidazolium, 2,3,13,14-tetrachloro-16,18-diethyl-7,7a,8a,9,10,16-hexahydro | 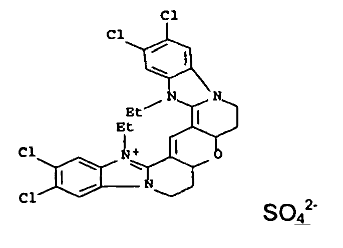 |
| Sel (par exemple chlorure) de 6H-Benzothiazolo[2,3-f]benzothiazolo[3',2': 1,2]pyrido[4,3-b][1,6]naphthyridin-5-ium, 8-(acetylamino)-7,7a,8,8a,9,10-hexahydro | 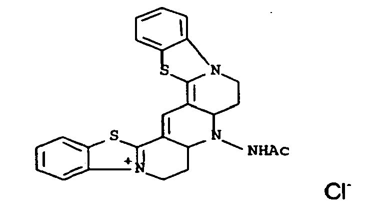 |
| Sel (par exemple chlorure) de 1H-Benzimidazo[2,1-f]benzimidazo[1',2':1,2]pyrido[4,3-b][1,6]naphthyridin-5-ium | 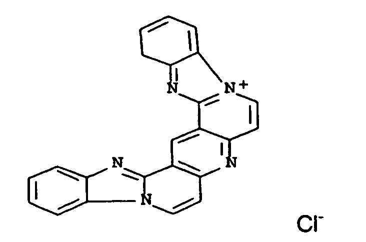 |
| Iodure de Benzimidazo[2,1-f] benzimidazo[1',2':1,2]pyrido[4,3-b][1,6]naphthyridinium, 2,3,13,14-tetrachloro-16,18-diethyl-6,7,7a,8,8a,9,10,16-octahydro-8-[(4-methylphenyl)amino]-, | 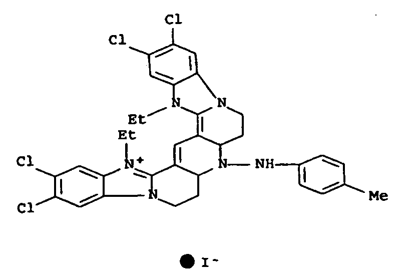 |
| Sel (par exemple chlorure) de Pyrano [3",2":3,4;5",6":3',4']dipyrido[1,2-a:1',2'-a']bisbenzimidazol-5- | 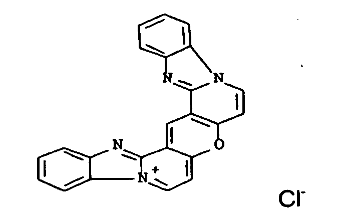 |
| Sel (par exemple chlorure) de 6H,10H-Pyrano[3",2":3,4;5",6":3',4']dipyrido[2,1-b:2',1'-b']bisbenzothiazol-5-ium | 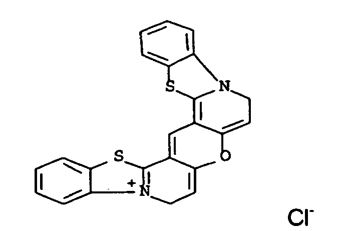 |
| Sel (par exemple chlorure) de 6H-Benzothiazolo[2,3-f]benzothiazolo[3',2': 1,2]pyrido[4,3-b][1,6]naphthyridin-5-ium | 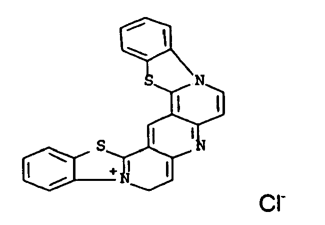 |
| Sel (par exemple chlorure) de 6H,10H-Pyrano[3",2":3,4;5",6":3',4']dipyrido[2,1-b:2',1'-b']bisbenzoxazol-5- | 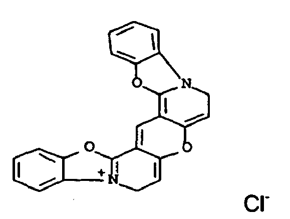 |
| Sel (par exemple chlorure) de 6H-Benzoxazolo[2,3-f]benzoxazolo[3',2':1,2]pyrido[4,3-b][1,6]naphthyridin-5-ium | 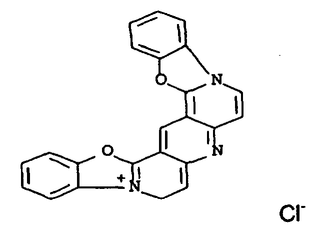 |
| Sel (par exemple chlorure) de 6H-Benzimidazo[2,1-f] benzimidazo[1',2':1,2]pyrido[4,3-b][1,6]naphthyridin-5-ium | 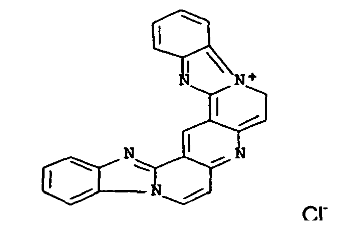 |
| Dichloroiodate 6H-Benzothiazolo[2,3-f]benzothiazolo[3',2':1,2]pyrido[4,3-b][1,6]naphthyridin-5-ium, 8-(acetylamino)-7,7a,8,8a,9,10-hexahydro-, | 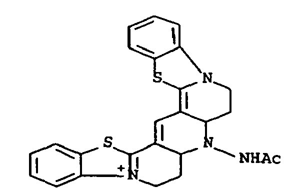 |
| Chlorure de 6,7,11,12-tetrahydro-9-oxo-9H-bisthiazolo[3,2-a:3',2'-a']-pyrimido[6,1-c:4,3-c']dipyrazin-5-ium | 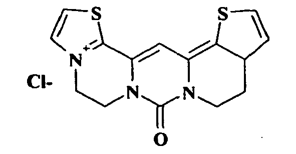 |
| Bromure de 6,7,11,12-tetrahydro-9-oxo-9H-bisoxazolo[3,2-a:3',2'-a']-pyrimido[6,1-c:4,3-c']dipyrazin-5-ium | 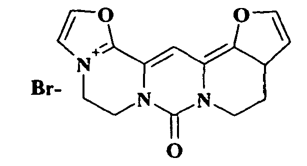 |
| Bromure de 6,7,11,12-tetrahydro-9-oxo-9H-bisselenazolo[3,2-a:3',2'-a']-pyrimido[6, 1-c:4,3-c']dipyrazin-5-ium | 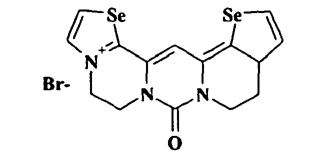 |

La nature du contre-ion n'est pas critique. Ainsi les anions mentionnés dans ce tableau ci-dessus ne sont donnés qu'à titre d'exemple.

Le ou les colorants directs de formules (I) ou (I') représentent plus particulièrement de 0,01 à 20 % en poids, de préférence de 0,1 à 5 % en poids par rapport au poids total de la composition.

Le milieu cosmétiquement acceptable est généralement constitué par de l'eau ou par un mélange d'eau et d'au moins un solvant organique.
A titre de solvant organique, on peut par exemple citer, les alcanols, linéaires ou ramifiés, en C₁-C₄, tels que l'éthanol et l'isopropanol ; le glycérol ; les glycols et éthers de glycols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Le ou les solvants peuvent être présents dans des proportions allant de préférence de 1 à 40 % en poids environ par rapport au poids total de la composition tinctoriale, et encore plus préférentiellement de 5 à 30 % en poids environ.

Le pH de la composition conforme à invention est généralement compris entre 3 et 12 environ, et de préférence entre 5 et 11 environ. Il peut être ajusté à la valeur désirée au moyen d'agents acidifiants ou alcalinisants habituellement utilisés dans le domaine.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 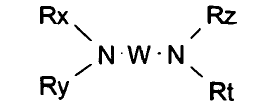 dans laquelle W est un reste propylène éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆.

La composition cosmétique peut comprendre en outre un ou plusieurs colorants directs additionnels de nature non ionique, cationique ou anionique, et de préférence cationique ou non ionique, ou leurs combinaisons.

Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthytoxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition mise en oeuvre dans le cadre de cette première variante peut également comprendre, en addition ou en remplacement de ces colorants benzéniques nitrés, un ou plusieurs colorants directs choisis parmi les colorants benzéniques nitrés jaunes, jaune-verts, bleus ou violets, les colorants azoïques, les colorants anthraquinoniques, naphtoquinoniques ou benzoquinoniques, les colorants indigoïdes, ou les colorants dérivés du triarylméthane.

Ces colorants directs peuvent notamment être des colorants basiques parmi lesquels on peut citer plus particulièrement les colorants connus dans le Color Index, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP 714954 et dont le contenu fait partie intégrante de la présente invention.

Parmi les colorants directs additionnels benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,y-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β**,**γ-dihydroxypropyl)amino 4-(N-éthyl, N-β-hydroxyéthyl)amino 2-nitrobenzène,
- les 2-nitroparaphénylènediamines de formule suivante : 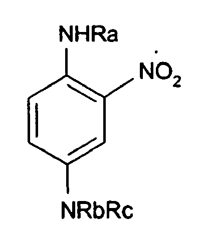
dans laquelle :
- R_{b} représente un radical alkyle en C₁-C₄, un radical β-hydroxyéthyle ou β-hydroxypropyle ou γ-hydroxypropyle ;
- Rₐ et R_{c}, identiques ou différents, représentent un radical β-hydroxyéthyle, -β-hydroxypropyle, γ-hydroxypropyle, ou β,γ-dihydroxypropyle, l'un au moins des radicaux R_{b}, R_{c} ou Rₐ représentant un radical γ-hydroxypropyle et R_{b} et R_{c} ne pouvant désigner simultanément un radical β-hydroxyéthyle lorsque R_{b} est un radical γ-hydroxypropyle, telles que celles décrits dans le brevet français FR 2 692 572.

Parmi les colorants directs naturels, on peut citer le henné, la camomille, l'indigo, entre autres.

Lorsqu'ils sont présents, le ou les colorants directs additionnels représentent de préférence de 0,0005 à 12 % en poids, et encore plus préférentiellement de 0,005 à 6 % en poids par rapport poids total de la composition.

Lorsqu'elle est destinée à la teinture d'oxydation, la composition cosmétique conforme à l'invention comprend de plus, au moins une base d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxy-méthyl paraphénylènediamine, la N,N-diméthyl 3-méthyl paraphénylènediamine, la N,N-(éthyl, β-hydroxyéthyl) paraphénylène diamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-(β-acétylaminoéthyloxy paraphénylènediamine, la N-(β-méthoxyéthyl) paraphénylène-diamine et la 4'aminophényl 1-(3hydroxy)pyrrolidine, et leurs sels d'addition avec un acide ou avec un agent alcalin.
Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou avec un agent alcalin.
Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylène-diamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple, les dérivés pyridiniques, les dérivés pyrimidiniques et les dérivés pyrazoliques et leurs sels d'addition avec un acide ou avec un agent alcalin.

Lorsqu'elles sont utilisées, la ou les bases d'oxydation représentent de préférence de 0,0005 à 12 % en poids, et encore plus préférentiellement de 0,005 à 6 % en poids, par rapport poids total de la composition.

Lorsqu'elle est destinée à la teinture d'oxydation, la composition selon l'invention peut également comprendre au moins un coupleur de façon à modifier ou à enrichir en reflets les nuances obtenues en mettant en oeuvre les colorants directs et la ou les bases d'oxydation.

Les coupleurs utilisables peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent de préférence de 0,0001 à 10 % en poids, et encore plus préférentiellement de 0,005 à 5 % en poids par rapport poids total de la composition.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.
Les sels d'addition avec un agent alcalin utilisables dans le cadre des compositions de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (II).

La composition cosmétique conforme à l'invention peut également comprendre divers adjuvants utilisés classiquement dans les compositions cosmétiques notamment de teinture des fibres kératiniques humaines, tels que des polymères anioniques, cationiques, non ioniques, amphotères, zwittérioniques ou leurs mélanges ; des agents épaississants minéraux ; des agents antioxydants ; des agents de pénétration ; des agents séquestrants ; des parfums ; des tampons ; des agents dispersants ; des agents de conditionnement tels que par exemple des cations, des polymères cationiques ou amphotères, les chitosanes, les silicones volatiles ou non volatiles, modifiées ou non modifiées ; des agents filmogènes ; des céramides ; des agents conservateurs ; des agents stabilisants ; des agents opacifiants.

Selon un mode de réalisation particulièrement avantageux, la composition comprend un ou plusieurs agents tensioactifs. Ces derniers peuvent être indifféremment choisis, seuls ou en mélanges, parmi des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de préférence de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

### (iii) Tensioactif(s) amphotère(s) ou zwittérionique(s) :

Les agents tensioactifs amphotères ou zwitterioniques peuvent être notamment choisis parmi les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C8-C20) bétaïnes, les sulfobétaïnes, les alkyl (C8-C20) amidoalkyl (C1-C6) betaïnes ou les alkyl (C8-C20) amidoalkyl (C1-C6) sulfobétaïnes.

Parmi les dérivés d'amines, on peut citer les produits vendus sous la dénomination MIRANOL, tels que décrits dans les brevets US 2528378 et US 2781354 et classés dans le dictionnaire CTFA, 3ème édition, 1982, sous les dénominations Amphocarboxyglycinates et Amphocarboxypropionates de structures respectives :
Rd -CONHCH2CH2 -N(Re)(Rf)(CH2COO⁻)
dans laquelle : Rd désigne un radical alkyle d'un acide Rd-COOH présent dans l'huile de coprah hydrolysée, un radical heptyle, nonyle ou undécyle, Re désigne un groupement bêta-hydroxyéthyle et Rf un groupement carboxyméthyle ; et
Rg-CONHCH2CH2-N(B)(C)
dans laquelle :
B représente -CH2CH2OX, C représente -(CH2)z -Y, avec z = 1 ou 2,
X désigne le groupement -CH2CH2-COOH ou un atome d'hydrogène
Y désigne -COOH ou le radical -CH2 - CHOH - SO3H
Rg désigne un radical alkyle d'un acide Rh -COOH présent dans l'huile de coprah ou dans l'huile de lin hydrolysée, un radical saturé ou comprenant une ou plusieurs insaturations, notamment en C7 à C17, plus particulièrement un radical alkyle en C9, C11, C13, C17 ou sa forme iso, un radical C17 insaturé.

Ces composés sont classés dans le dictionnaire CTFA, 5ème édition, 1993, sous les dénominations Disodium Coco-ampho-diacetate, Disodium Lauro-ampho-diacetate, Disodium Capryl-ampho-diacetate, Disodium Caprylo-ampho-diacetate, Disodium Coco-ampho-dipropionate, Disodium Lauro-ampho-dipropionate, Disodium Capryl-ampho-dipropionate, Disodium Caprylo-ampho-dipropionate, Lauro-ampho-dipropionic acid, Coco-ampho-dipropionic acid.
A titre d'exemple on peut citer le cocoamphodiacetate commercialisé sous la dénomination commerciale Miranol^{®} C2M concentré par la société Rhodia Chimie.

### (iv) Tensioactifs cationiques :

Parmi les tensioactifs cationiques on peut citer en particulier les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

De préférence, les tensioactifs sont non ioniques, anioniques ou amphotères et de manière encore plus préférée, non ioniques.

Habituellement, les agents tensioactifs sont présents dans une quantité comprise entre 0,01 et 50 % en poids, de préférence entre 0,1 et 25 % en poids par rapport au poids total de la composition.

La composition peut de plus comprendre un ou plusieurs polymères épaississants. Ces polymères peuvent être ioniques ou non, associatifs ou non.

En ce qui concerne les polymères épaississants non associatifs, il est tout d'abord rappelé qu'au sens de la présente invention, les polymères épaississants non associatifs sont des polymères épaississants ne contenant pas de chaîne grasse en C₁₀-C₃₀.

Parmi les polymères épaississants non associatifs présents, on peut citer les homopolymères d'acide acrylique réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide, les gommes de guar non ioniques, les gommes de biopolysaccharides d'origine microbienne, les gommes issues d'exudats végétaux, les hydroxypropyl- ou carboxyméthyl- celluloses ; les pectines et les alginates, seuls ou en mélanges.

Une première famille de polymères épaississants non associatifs convenable est représentée par les homopolymères d'acide acrylique réticulés. Parmi les homopolymères de ce type, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

Les polymères épaississants non associatifs peuvent aussi être choisis parmi les homopolymères réticulés d'acide 2-acrylamido-2-méthylpropane sulfonique et leurs copolymères réticulés d'acrylamide.

En ce qui concerne ces homopolymères et copolymères, qui peuvent être partiellement ou totalement neutralisés, on peut citer les polymères comprenant de 90 à 99,9% en poids, par rapport au poids total du polymère, de motifs de formule (j) suivante : 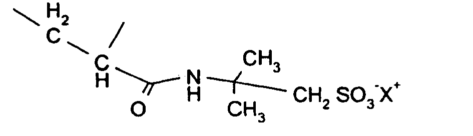 dans laquelle X⁺ désigne un cation ou un mélange de cations, ou un proton.

Plus particulièrement les cations sont choisis parmi les métaux alcalins (comme le sodium, le potassium), les ions ammonium substitués ou non par un à trois radicaux alkyle, identiques ou différents, comprenant 1 à 6 atomes de carbone, éventuellement porteur d'au moins un radical hydroxyle, les cations dérivant de la N-méthyl-glucamine, d'acides aminés basiques comme l'arginine et la lysine. De préférence, le cation est un ion ammonium ou sodium.

Par ailleurs, le polymère comprend de 0,01 à 10% en poids, par rapport au poids total du polymère, de motifs réticulants provenant d'au moins un monomère ayant au moins deux insaturation éthyléniques (double liaison carbone-carbone).
Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Pour plus de détail au sujet de ces polymères, on pourra se reporter au document EP 815828.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

La composition peut de même comprendre, à titre de polymères épaississants non associatifs, les homopolymères d'acrylate d'ammonium ou les copolymères d'acrylate d'ammonium et d'acrylamide. A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

La composition peut aussi comprendre des homopolymères de diméthylaminoéthylméthacrylate quaternisé par le chlorure de méthyle ou les copolymères de diméthylaminoéthylméthacrylate quaternisé par le chlorure de méthyle et d'acrylamide.
Parmi les homopolymères de ce type, on peut citer les produits vendus sous les noms SALCARE 95 et SALCARE 96 par la société CIBA-ALLIED COLLOIDS. Parmi les copolymères de cette famille, on peut citer le produit SALCARE SC92 vendu par CIBA-ALLIED COLLOIDS ou le produit PAS 5194 vendu par HOECHST. Ces polymères sont notamment décrits et préparés dans le document EP 395282 auquel on pourra se référer.

La composition peut aussi comprendre des gommes de guar non ioniques, comme par exemple les gommes de guar non ioniques non modifiées vendues sous la dénomination VIDOGUM GH 175 par la société UNIPECTINE et sous la dénomination JAGUAR C par la société MEYHALL.
Les gommes de guar non ioniques utilisables selon l'invention sont de préférence modifiées par des groupements hydroxyalkyle en C₁-C₆. Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent par exemple être préparées en faisant réagir des oxydes d'alcènes correspondants, tels que par exemple des oxydes de propylène, avec la gomme de guar, de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2 et.
De telles gommes de guar non ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL ou sous la dénomination GALACTASOL 4H4FD2 par la société AQUALON.

A titre de polymères épaississants non associatifs convenables, on peut aussi mentionner les gommes de biopolysaccharides d'origine microbienne telles que les gommes de scléroglucane ou de xanthane.
Conviennent aussi les gommes issues d'exudats végétaux, telles que les gommes arabiques, gommes Ghatti, gommes Karaya et Tragacanthe ; les hydroxypropyl- ou carboxyméthyl- celluloses ; les pectines et les alginates.
Ces polymères sont bien connus de l'homme de l'art et sont notamment décrits dans l'ouvrage de Robert L. DAVIDSON intitulé "Handbook of Water soluble gums and resins" édité chez Mc Graw Hill Book Company (1980).

Parmi les agents épaississants, on préfère plus particulièrement utiliser les systèmes épaississants à base de polymères associatifs bien connus de l'homme de l'art et notamment de nature non ionique, anionique, cationique ou amphotère.

Il est rappelé que les polymères associatifs sont des polymères hydrophiles capables, dans un milieu aqueux, de s'associer réversiblement entre eux ou avec d'autres molécules. Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone. Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

La composition peut donc comprendre au moins un polymère associatif choisi parmi les polyuréthanes associatifs, plus particulièrement cationiques ou non ioniques, les dérivés de cellulose associatifs, plus particulièrement cationiques ou non ioniques, les vinyllactames associatifs, les polyacides insaturés associatifs, les aminoplaste-éthers associatifs, les polymères ou copolymères associatifs comprenant au moins un monomère à insaturation éthylénique à groupement sulfonique, seuls ou en mélanges.

Parmi les polymères épaississants associatifs, on peut citer les dérivés de polyuréthanes associatifs, comme ceux obtenus par polymérisation :
- environ 20% à 70% en poids d'un acide carboxylique à insaturation α,β-monoéthylénique,
- environ 20 à 80% en poids d'un monomère à insaturation α,β-monoéthylénique non-tensioactif différent du précédent,
- environ 0,5 à 60% en poids d'un mono-uréthane non-ionique qui est le produit de réaction d'un tensioactif monohydroxylé avec un monoisocyanate à insaturation monoéthylénique. De tels sont notamment décrits dans EP 173109 et plus particulièrement dans l'exemple 3. Plus précisément, ce polymère est un terpolymère acide méthacrylique / acrylate de méthyle / diméthyl métaisopropényl benzyl isocyanate d'alcool béhényle éthoxylé (40OE) en dispersion aqueuse à 25%.Ce produit est proposé sous la référence VISCOPHOBE DB1000 par la Société AMERCHOL.

Conviennent aussi les polyuréthanes associatifs cationiques dont la famille a été décrite dans la demande FR 0009609. Elle peut être représentée plus particulièrement par la formule générale (A) suivante :

R-X-(P)ₙ-[L-(Y)ₘ]ᵣ-L'-(P')ₚ-X'-R' (A)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ; P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25 ;
n, m, et p valent chacun indépendamment des autres entre 0 et 1000 ;
la molécule contenant au moins une fonction amine protonée ou quaternisée et au moins un groupement hydrophobe.

Dans un mode de réalisation très avantageux, les seuls groupements hydrophobes de ces polyuréthanes sont les groupes R et R' situés aux extrémités de chaîne.

Selon un premier mode de réalisation préféré, le polyuréthane associatif correspond à la formule (A) dans laquelle R et R' représentent tous les deux indépendamment un groupement hydrophobe ; X, X' représentent chacun un groupe L" ; n et p valent entre 1 et 1000 et L, L', L", P, P', Y et m ont la signification indiquée que dans la formule (A).

Selon un autre mode de réalisation préféré de l'invention, le polyuréthane associatif correspond à la formule (A) dans laquelle R et R' représentent tous les deux indépendamment un groupement hydrophobe, X, X' représentent chacun un groupe L", n et p valent 0, et L, L', L", Y et m ont la signification que dans la formule (A) indiquée auparavant.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Conformément à un autre mode de réalisation préféré de l'invention, le polyuréthane associatif correspond à la formule (A) dans laquelle R et R' représentent tous les deux indépendamment un groupement hydrophobe ; X et X' représentent tous les deux indépendamment un groupement comportant une amine quaternaire ; n et p valent zéro, et L, L', Y et m ont la signification indiquée dans la formule (A).

La masse moléculaire moyenne en nombre des polyuréthanes associatifs cationiques est habituellement comprise entre 400 et 500000, en particulier entre 1000 et 400000 et idéalement entre 1000 et 300000 g/mol.

Lorsque X et/ou X' désignent un groupement comportant une amine tertiaire ou quaternaire, X et/ou X' peuvent représenter l'une des formules suivantes : 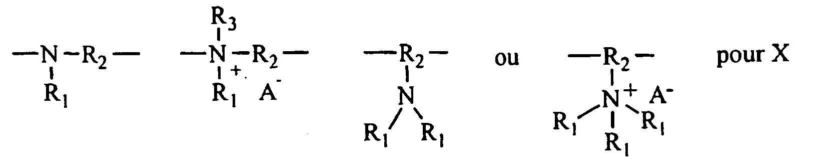 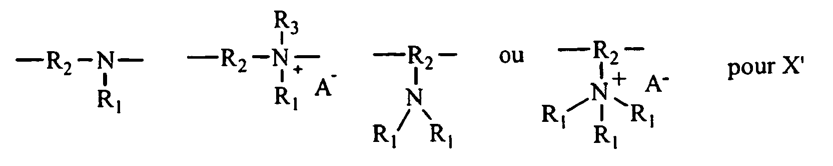 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 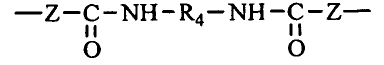 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 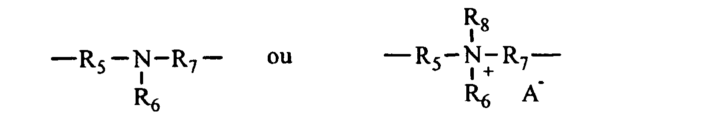 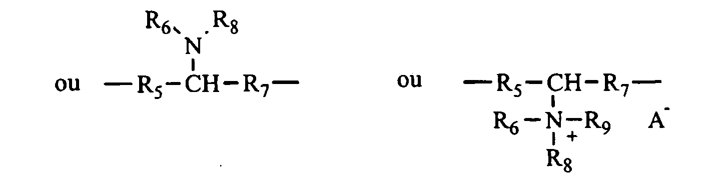 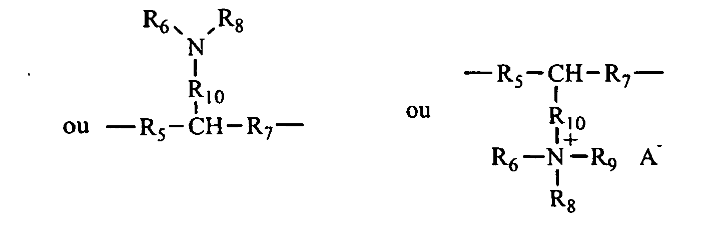 dans lesquelles :
R₅ et R₇ ont les mêmes significations que R₂ défini précédemment ;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P ;
A⁻ est un contre-ion cosmétiquement acceptable.

En ce qui concerne la signification de Y, on entend par groupement hydrophile, un groupement hydrosoluble polymérique ou non. A titre d'exemple, on peut citer, lorsqu'il ne s'agit pas de polymères, l'éthylèneglycol, le diéthylèneglycol et le propylèneglycol. Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs de formule (A) sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (A) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine.
Ce type de composés peut être représenté par l'une des formules suivantes :
HZ-(P)ₙ-ZH, ou HZ-(P')ₚ-ZH
dans lesquelles Z, P, P', n et p sont tels que définis plus haut.
A titre d'exemples de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (A) est un diisocyanate correspondant à la formule O=C=N-R₄-N=C=O, dans laquelle R₄ est défini plus haut.
On peut citer notamment le méthylènediphényl-diisocyanate, le méthylène cyclohexane diisocyanate, l'isophorone-diisocyanate, le toluènediisocyanate, le naphtalène diisocyanate, le butanediisocyanate, l'hexanediisocyanate.

Un troisième composé entrant dans la préparation du polyuréthane de formule (A) est un composé hydrophobe destiné à former les groupes hydrophobes terminaux du polymère de formule (A).
Ce composé est constitué d'un groupe hydrophobe et d'une fonction à hydrogène labile, par exemple une fonction hydroxyle, amine primaire ou secondaire, ou thiol.
A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné α-hydroxyle.
Le groupe hydrophobe du polyuréthane de formule (A) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire,
ou thiol. Ce composé peut être un polymère terminé aux extrémités des chaînes par l'une de ces fonctions à hydrogène labile.

A titre d'exemples, on peut citer, lorsqu'il ne s'agit pas de polymères, l'éthylèneglycol, le diéthylèneglycol et le propylèneglycol.
Lorsqu'il s'agit d'un polymère hydrophile, on peut citer à titre d'exemple les polyéthers, les polyesters sulfonés, les polyamides sulfonés, ou un mélange de ces polymères.
A titre préférentiel, le composé hydrophile est un polyéther et notamment un poly(oxyde d'éthylène) ou poly(oxyde de propylène).

Le groupe hydrophile noté Y dans la formule (A) est facultatif. En effet, les motifs à fonction amine quaternaire ou protonée peuvent suffire à apporter la solubilité ou l'hydrodispersibilité nécessaire pour ce type de polymère dans une solution aqueuse.
Bien que la présence d'un groupe Y hydrophile soit facultative, on préfère cependant des polyuréthanes associatifs cationiques comportant un tel groupe.

Les dérivés de polyuréthanes associatifs de l'invention peuvent être aussi des polyuréthanes polyéthers non ioniques. Plus particulièrement, lesdits polymères comportent dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

Les polyéthers polyuréthanes non ioniques comportent une liaison uréthane entre les séquences hydrophiles, d'où l'origine du nom.
Par extension figurent aussi parmi les polyéthers polyuréthanes non ioniques à chaîne hydrophobe, ceux dont les séquences hydrophiles sont liées aux séquences hydrophobes par d'autres liaisons chimiques.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.
On peut également citer le produit ELFACOS T210® à chaîne alkyle en C₁₂-C₁₄ et le produit ELFACOS T212® à chaîne alkyle en C₁₈ de chez AKZO.
Le produit DW 1206B® de chez ROHM & HAAS à chaîne alkyle en C₂₀ et à liaison uréthane, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.
Les polyéthers polyuréthanes utilisables décrits auparavant peuvent aussi être choisis parmi ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.
De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

La composition peut de même comprendre des polymères dérivés de celluloses associatifs tels que :
- les celluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci,
- les hydroxyéthylcelluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.
   Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
   On peut indiquer comme exemples d'alkylhydroxyéthylcelluloses quaternisées à chaîne hydrophobe en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.
- les dérivés de cellulose non ioniques tels que les hydroxyéthylcelluloses modifiées par des groupements comportant au moins une chaîne hydrophobe tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
- les dérivés de cellulose modifiée par des groupes polyalkylène glycol éther d'alkyl phénol, tel que le produit AMERCELL POLYMER HM-1500® vendu par la société AMERCHOL.

En ce qui concerne les polyvinyllactames associatifs, on peut citer par exemple les polymères notamment décrits dans FR 0101106. Lesdits polymères sont plus particulièrement des polymères cationiques et comprennent :
-a) au moins un monomère de type vinyl lactame ou alkylvinyllactame;
-b) au moins un monomère de structures (a) ou (b) suivantes : 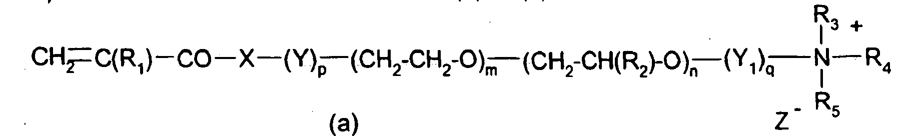 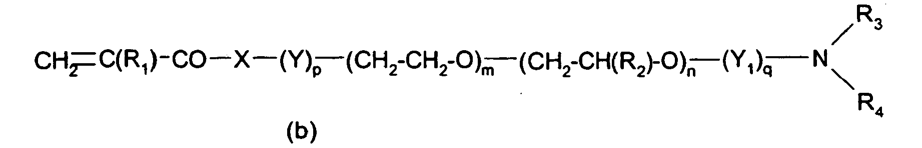
dans lesquelles :
X désigne un atome d'oxygène ou un radical NR6,
R1 et R6 désignent, indépendamment l'un de l'autre, un atome d'hydrogène ou un radical alkyl linéaire ou ramifié en C1-C5,
R2 désigne un radical alkyle linéaire ou ramifié en C1-C4,
R3, R4 et R5 désignent, indépendamment l'un de l'autre, un atome d'hydrogène, un radical alkyle linéaire ou ramifié en C1-C30 ou un radical de formule (IV) :

   -(Y₂)ᵣ-(CH₂-CH(R₇)-O)ₓ-R₈ (c)
Y, Y1 et Y2 désignent, indépendamment l'un de l'autre, un radical alkylène linéaire ou ramifié en C2-C16,
R7 désigne un atome d'hydrogène, ou un radical alkyle linéaire ou ramifié en C1-C4 ou un radical hydroxyalkyle linéaire ou ramifié en C1-C4,
R8 désigne un atome d'hydrogène ou un radical alkyle linéaire ou ramifié en C1-C30,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
   sous réserve que :
   - un au moins des substituants R3, R4, R5 ou R8 désigne un radical alkyle linéaire
   ou ramifié en C9-C30,
   - si m ou n est différent de zéro, alors q est égal à 1,
   - si m ou n sont égaux à zéro, alors p ou q est égal à 0.

Les polymères poly(vinyllactame) peuvent être réticulés ou non réticulés et peuvent aussi être des polymères blocs.

De préférence le contre ion Z⁻ des monomères de formule (b) est choisi parmi les ions halogénures, les ions phosphates, l'ion méthosulfate, l'ion tosylate.

De préférence R3, R4 et R5 désignent, indépendamment l'un de l'autre, un atome d'hydrogène ou un radical alkyle linéaire ou ramifié en C1-C30.
Plus préférentiellement, le monomère b) est un monomère de formule (b) pour laquelle, encore plus préférentiellement, m et n sont égaux à zéro.

Le monomère vinyllactame ou alkylvinyllactame est de préférence un composé de structure (d) : 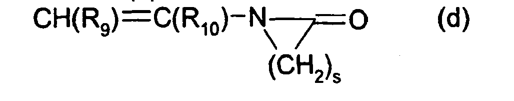 dans laquelle :
s désigne un nombre entier allant de 3 à 6,
R9 désigne un atome d'hydrogène ou un radical alkyle en C1-C5,
R10 désigne un atome d'hydrogène ou un radical alkyle en C1-C5,
sous réserve que l'un au moins des radicaux R9 et R10 désigne un atome d'hydrogène.

Encore plus préférentiellement, le monomère (d) est la vinylpyrrolidone.

Les polymères poly(vinyllactame) peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (d),
b)-un monomère de formule (a) dans laquelle p=1, q=0, R3 et R4 désignent, indépendamment l'un de l'autre, un atome d'hydrogène ou un radical alkyle en C1-C5 et R5 désigne un radical alkyle en C9-C24 et
c)-un monomère de formule (b) dans laquelle R3 et R4 désignent, indépendamment l'un de l'autre, un atome d'hydrogène ou un radical alkyle en C1-C5.

Encore plus préférentiellement, on met en oeuvre des terpolymères comprenant, en poids, 40 à 95% de monomère (d), 0,1 à 55% de monomère (b) et 0,25 à 50% de monomère (b).
De tels polymères sont notamment décrits dans la demande de brevet WO 00/68282 dont le contenu fait partie intégrante de l'invention.

Comme polymères poly(vinyllactame), on utilise notamment les terpolymères vinylpyrrolidone / diméthylaminopropylméthacrylamide / tosylate de dodécyl diméthylméthacrylamidopropylammonium, les terpolymères vinylpyrrolidone / diméthyl aminopropylméthacrylamide / tosylate de cocoyldiméthylméthacrylamido propyl ammonium, les terpolymères vinylpyrrolidone / diméthylaminopropyl méthacrylamide /tosylate ou chlorure de lauryldiméthylméthacrylamidopropylammonium. Le terpolymère vinylpyrrolidone / diméthylaminopropyl méthacrylamide /chlorure de lauryl diméthylméthacrylamidopropylammonium est proposé dans l'eau à 20% par la Société ISP sous la dénomination STYLEZE W20.

Les dérivés de polyvinyllactames associatifs de l'invention peuvent être aussi des copolymères non ioniques de vinylpyrrolidone et de monomères hydrophobes à chaîne hydrophobe dont on peut citer à titre d'exemple :
- les produits ANTARON V216® ou GANEX V216® (copolymère vinylpyrrolidone /hexadécène) vendu par la société I.S.P.
- les produits ANTARON V220® ou GANEX V220® (copolymère vinylpyrrolidone /eicosène) vendu par la société I.S.P.

Parmi les dérivés de polyacides insaturés associatifs on peut citer ceux comportant au moins un motif hydrophile de type acide carboxylique insaturé oléfinique, et au moins un motif hydrophobe de type ester d'alkyl (C10-C30) d'acide carboxylique insaturé.
Ces polymères sont notamment choisis parmi ceux dont le motif hydrophile de type acide carboxylique insaturé oléfinique correspond au monomère de formule (e) suivante : 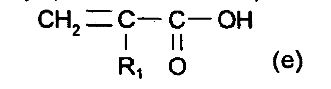 dans laquelle, R1 désigne H ou CH3 ou C2H5, c'est-à-dire des motifs acide acrylique, acide méthacrylique ou acide éthacrylique, et dont le motif hydrophobe de type ester d'alkyl (C10-C30) d'acide carboxylique insaturé correspond au monomère de formule (f) suivante : 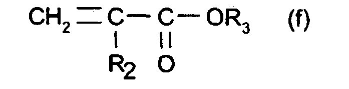 dans laquelle, R₂ désigne H ou CH3 ou C2H5 (c'est-à-dire des motifs acrylates, méthacrylates ou éthacrylates) et de préférence H (motifs acrylates) ou CH3 (motifs méthacrylates), R₃ désignant un radical alkyle en C10-C30, et de préférence en C12-C22. Des esters d'alkyle (C10-C30) d'acides carboxyliques insaturés comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
Des polymères anioniques de ce type sont par exemple décrits et préparés, selon les brevets US-3 915 921 et 4 509 949.

Dans ce type de polymères associatifs anioniques, on utilise plus particulièrement des polymères formés à partir d'un mélange de monomères comprenant :
(i) essentiellement de l'acide acrylique,
(ii) un ester de formule (f) décrite ci-dessus et dans laquelle R2 désigne H ou CH3, R3 désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on préfère ceux constitués de 95 à 60% en poids d'acide acrylique (motif hydrophile), de 4 à 40% en poids d'acrylate d'alkyles en C₁₀-C₃₀ (motif hydrophobe), et de 0 à 6% en poids de monomère polymérisable réticulant, ou bien ceux constitués de 98 à 96% en poids d'acide acrylique (motif hydrophile), de 1 à 4% en poids d'acrylate d'alkyles en C₁₀-C₃₀ (motif hydrophobe), et de 0,1 à 0,6% en poids de monomère polymérisable réticulant tel que ceux décrits précédemment.

Parmi lesdits polymères ci-dessus, on préfère tout particulièrement les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

Parmi les dérivés de polyacides insaturés associatifs on peut aussi citer ceux comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C1-C4.
A titre d'exemples de ce type de composés, on peut citer l'ACULYN 22® vendu par la société ROHM et HAAS, qui est un terpolymère acide méthacrylique/acrylate d'éthyle/méthacrylate de stéaryle oxyalkyléné.

En ce qui concerne les polymères épaississants du type des aminoplaste-éther, on désigne tout produit issu de la condensation d'un aldéhyde avec une amine ou un amide, ainsi que toute unité structurale formée d'un résidu aminoplaste et d'un résidu hydrocarboné divalent lié au résidu aminoplaste par une liaison éther.

Les polymères à squelette aminoplaste-éther sont choisis de préférence parmi ceux contenant au moins un motif de structure (g) suivante : 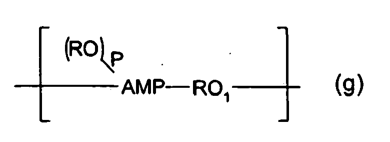 dans laquelle:
- AMP est un résidu aminoplaste avec des unités alkylènes (ou alkyle divalent),
- R désigne un atome d'hydrogène, un radical alkyle C₁-C₄ ou un radical acyle C₁-C₄,
- RO₁ est un résidu alkylène-oxy divalent,
- p désigne un nombre entier positif,
- le ou les groupements OR étant liés aux unités alkylènes du résidu AMP.

De préférence, les polymères à squelette aminoplaste-éther sont choisis parmi ceux contenant au moins un motif de structure (h) suivante : 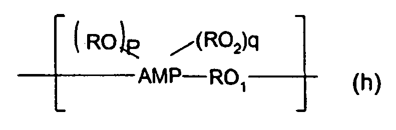 dans laquelle :
- AMP, R, RO₁ et p ont la même signification que précédemment,
- RO₂ est un groupe différent de RO lié à AMP au moyen d'un hétéroatome et comprenant au moins deux atomes de carbone, et,
- q est un nombre entier positif.

Plus préférentiellement encore, les polymères correspondent aux formules (III) et (III)bis suivantes : 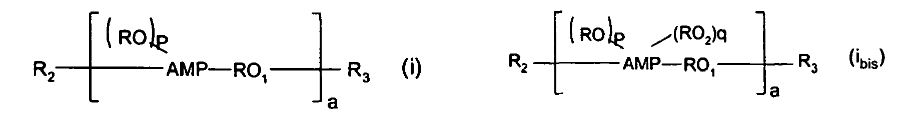 dans lesquelles :
- AMP, R, RO₁, RO₂, p et q ont la même signification que précédemment,
- R₂ ou R₃, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
- a étant un nombre supérieur à 1 et de préférence supérieur à 2.

Les résidus aminoplastes porteurs de leurs groupements OR intégrés dans les polymères peuvent être choisis de manière non limitative parmi les structures (1) à (12) suivantes : 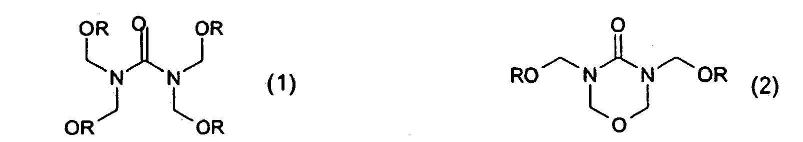 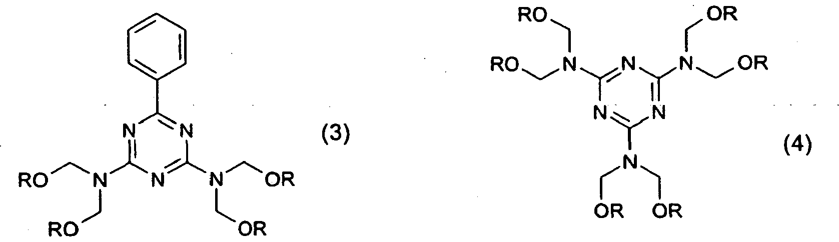 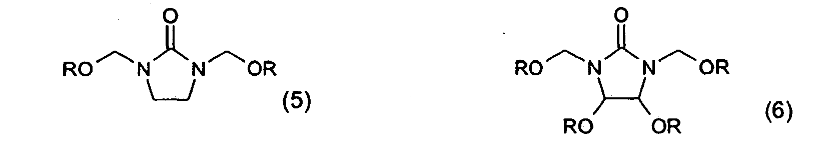 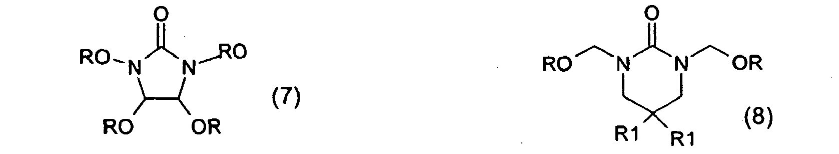 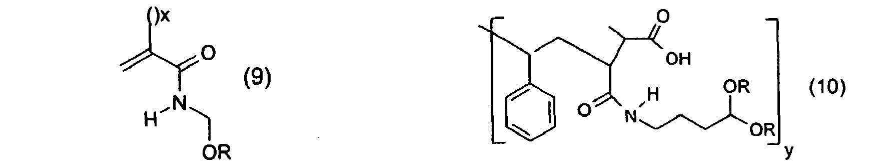 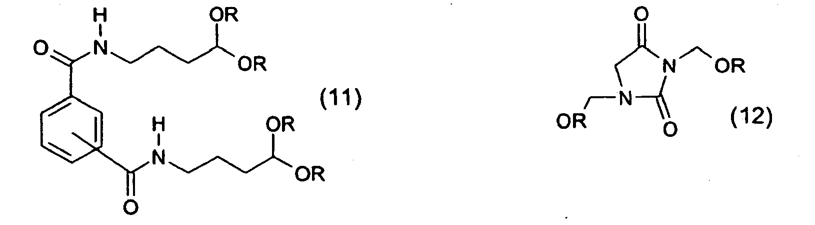 dans lesquelles :
- R a la même signification que précédemment,
- R₁ désigne alkyle C₁-C₄,
- y est un nombre au moins égal à 2,
- x désigne 0 ou 1.

De préférence, le ou les résidus aminoplastes porteurs de leurs groupements OR sont choisis parmi ceux de structure (13) suivante : 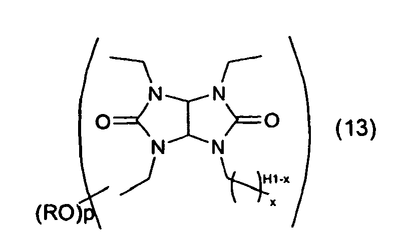 dans laquelle R, p , et x ont les mêmes significations que précédemment.

Les résidus alkylène-oxy divalents sont de préférence ceux correspondants aux diols de formule générale (14) suivante :

HO-(ZO)_{y}-(Z₁(Z₂O)_{w})ₜ-(Z'O)y'-Z₃OH (14),

- y et y' étant des nombres allant de 0 à 1000,
- t et w étant des nombres allant de 0 à 10,
- Z, Z', Z₂ et Z₃ sont des radicaux alkylène en C₂-C₄ et de préférence des radicaux
- CH₂-CH(Z₄)- et -CH₂-CH(Z₄)-CH₂-,
- Z₁ étant un radical linéaire ou cyclique, ramifié ou non, aromatique ou non, comportant ou non un ou plusieurs hétéroatomes et possédant de 1 à 40 atomes de carbone,
- Z₄ désignant un atome d'hydrogène ou un radical alkyle en C₁-C₄ ou un radical acyle en C₁-C₃ étant entendu qu'au moins un des radicaux Z₄ des radicaux Z, Z', Z₂ et Z₃ est différent d'un radical acyle.
De préférence Z₄ désigne un atome d'hydrogène ou un radical méthyle.
Encore plus préférentiellement t=0 et Z, Z' et Z₃ désignent -CH₂CH₂-, et l'un au moins de y ou y' est différent de 0. Les composés de formule (14) sont alors des polyéthylèneglycols.

Les polymères aminoplaste éther de formule (g) sont en particulier décrits dans le brevet US 5 914 373 auquel on pourra se référer pour plus de détails.
Comme polymères à squelette aminoplaste-éther de formule (g), on peut en particulier citer les produits Pure-Thix^{®} L [PEG-180/Octoxynol-40/TMMG Copolymer (Nom INCl)], Pure-Thix M^{®} [PEG-180/Laureth-50/TMMG Copolymer (Nom INCl)], Pure-Thix^{®} HH [Polyether-1 (Nom INCI)] ; Pure Thix TX-1442^{®} [copolymère PEG-18 / dodoxynol-5 / PEG-25 tristyrylphénol / tétraméthoxy méthyl glycolurile] proposés par la société Süd-Chemie.

Les polymères épaississants entrant comme ingrédient dans la composition selon l'invention peuvent aussi être choisis parmi les polymères associatifs comportant au moins un monomère à insaturation éthylénique à groupement sulfonique, sous forme libre
ou partiellement ou totalement neutralisée et comprenant au moins une partie hydrophobe.
De façon préférentielle, lesdits polymères sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Ces polymères associatifs peuvent être ou non réticulés, et de préférence sont des polymères réticulés. Dans ce cas, les agents réticulants provenant d'au moins un monomère ayant au moins deux insaturation éthyléniques (double liaison carbone-carbone).
Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.
On utilise plus particulièrement le méthylène-bis-acrylamide, le méthacrylate d'allyle ou le triméthylol propane triacrylate. Le taux de réticulation varie en général de 0,01 à 10% en mole, par rapport au polymère.
Les monomères à insaturation éthylénique à groupement sulfonique sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.
Plus particulièrement, on peut utiliser les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.
On utilise de préférence l'acide 2-acrylamido-2-méthylpropane-sulfonique (AMPS) ainsi que ses formes partiellement ou totalement neutralisées.

Les polymères amphiphiles présents dans la composition selon l'invention peuvent aussi être choisis parmi les polymères amphiphiles statistiques d'AMPS modifiés par réaction avec une n-monoalkylamine ou une di-n-alkylamine en C₆-C₂₂, et tels que ceux décrits dans la demande WO00/31154.

Les monomères hydrophobes qui constituent la partie hydrophobe du polymère sont choisis de préférence parmi les acrylates ou les acrylamides de formule (k) suivante : 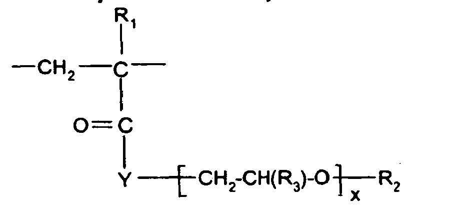 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe tel que défini auparavant ; x désigne un nombre de moles d'oxyde d'alkylène et varie de 0 à 100.

Le radical R₂ est choisi avantageusement parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulière de l'invention, le monomère de formule (k) comporte au moins un motif oxyde d'alkylène (x≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Les copolymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono-ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans les documents EP750899, US 5089578, les publications de Yotaro Morishima suivantes : «Self-assembling amphiphilic polyelectrolytes and their nanostructures - Chinese Journal of Polymer Science Vol. 18, N°40, (2000), 323-336.» ; «Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704» ; «Solution properties of miscelle networks formed by non-ionic moieties covalently bound to an polyelectrolyte: salt effects on rheological behaviour-Langmuir, 2000, Vol.16, N°12, 5324-5332» ; «Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

La répartition des monomères dans le copolymère peut être statistique ou bloc.

Parmi ces polymères de ce type, on peut citer plus spécialement :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578.
- les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs AMPS de formule (I) suivante : 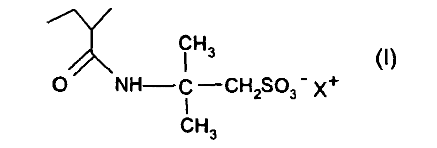 dans laquelle X⁺ a la même définition que précédemment,
et de motifs de formule (I) suivante : 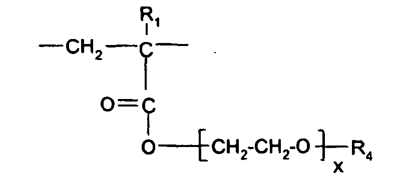 dans laquelle x désigne un nombre entier variant de 3 à 100, de préférence de 5 à 80 et plus préférentiellement de 7 à 25 ; R₁ a la même signification que celle indiquée ci-dessus dans la formule (j) et R₄ désigne un alkyle linéaire ou ramifié en C₆-C₂₂ et plus préférentiellement en C₁₀-C₂₂.

Les polymères particulièrement préférés sont ceux pour lesquels x = 25, R₁ désigne méthyle et R₄ représente n-dodécyle ; ils sont décrits dans les articles de Morishima mentionnés ci-dessus.
Les polymères pour lesquels X⁺ désigne sodium ou ammonium sont plus particulièrement préférés.
Les polymères de la gamme Genapol^{®} de la société Hoechst/Clariant peuvent être employés dans la composition selon l'invention.

La concentration en polymère(s) épaississant(s), associatif(s) ou non, présents dans la composition selon l'invention peut varier entre 0,01 et 10% en poids, plus particulièrement entre 0,1 à 5% en poids, par rapport au poids de la composition, et de manière encore plus avantageuse, entre 0,5 et 5 % en poids par rapport au poids de la composition.

Selon un mode de réalisation particulier, la composition selon l'invention comprend un tensio-actif et/ou un polymère épaississant.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de liquides, de shampooings, de crèmes, de gels, ou sous toute autre forme appropriée.

Une forme particulièrement préférée selon la présente invention est un shampooing colorant et/ou éclaircissant comprenant dans un milieu aqueux cosmétiquement acceptable, au moins un colorant direct tel que défini ci- dessus, et au moins un agent tensioactif de préférence non ionique.
Les agents tensioactifs non ioniques plus particulièrement préférés sont choisis parmi les alkylpolyglucosides.

Il n'est pas exclu, même si cela ne correspond pas à un mode de réalisation préféré de l'invention, que la composition renferme au moins un agent oxydant choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

Un autre objet de l'invention est constitué par un procédé de traitement de fibres kératiniques, en particulier de fibres kératiniques humaines.

Selon une première variante, on applique sur lesdites fibres, sèches ou humides, une composition telle que définie, pendant un temps suffisant, après quoi on rince, on lave éventuellement au shampooing, on rince à nouveau et on sèche ou on laisse sécher les fibres résultantes.

Selon une deuxième variante du procédé, on applique sur lesdites fibres, sèches ou humides, une composition telle que définie sans rinçage final.

La première variante est utilisable pour tout type de compositions, que celles-ci comprennent ou non, un agent oxydant et/ou un colorant direct et/ou une base d'oxydation éventuellement associée à un coupleur.

La seconde variante est particulièrement appropriée pour des compositions ne comprenant pas de colorant d'oxydation (base d'oxydation et éventuellement coupleur) ni d'agent oxydant.

Dans le cas de la première variante du procédé, le temps d'application est habituellement suffisant pour développer la coloration et/ou l'éclaircissement souhaité(s).
A titre indicatif, la durée d'application de la composition est d'environ 5 à 60 minutes et plus particulièrement d'environ 15 à 60 minutes.

Par ailleurs, la température à laquelle le procédé selon l'invention est mis en oeuvre, est généralement comprise entre la température ambiante (15 à 25°C) et 60°C et plus particulièrement entre 15 et 45°C.

Au cas où la composition comprend un agent oxydant, le procédé selon l'invention comporte une étape préliminaire consistant à stocker sous forme séparée, d'une part, une composition comprenant, dans un milieu cosmétiquement acceptable, au moins un colorant direct de formules (I) ou (I'), éventuellement au moins un colorant direct additionnel et/ou éventuellement au moins une base d'oxydation éventuellement associée à au moins un coupleur, et d'autre part, une composition comprenant, dans un milieu cosmétiquement acceptable, au moins un agent oxydant, puis à procéder à leur mélange au moment de l'emploi. Une fois ceci réalisé, le procédé selon l'invention est mis en oeuvre conformément à ce qui a été mentionné auparavant.

Un autre objet de l'invention est un dispositif à plusieurs compartiments, comprenant au moins un compartiment renfermant une composition comprenant au moins un colorant direct de formules (I) ou (I'), et au moins un autre compartiment renfermant une composition comprenant au moins un agent oxydant. Ce dispositif peut être équipé d'un moyen permettant de délivrer sur les cheveux le mélange souhaité, tel que les dispositifs décrits dans le brevet FR 2586913.
A noter qu'au cas où la composition renferme au moins un colorant direct additionnel et/ou au moins une base d'oxydation éventuellement associée à au moins un coupleur, selon une première variante, ce ou ces composés se trouvent dans le premier compartiment du dispositif précédemment décrit. Selon une deuxième variante, le colorant direct additionnel et/ou la base d'oxydation/coupleur sont stockés dans un troisième compartiment.
Il est précisé qu'il ne serait pas exclu d'avoir une troisième variante combinant les deux précédentes, dans laquelle le colorant direct additionnel et/ou la base d'oxydation et éventuellement le coupleur se trouveraient en partie dans le premier compartiment, avec le composé direct de formules (I) ou (I'), et en partie dans un troisième compartiment.

Les exemples qui suivent sont destinés à illustrer l'invention sans pour autant en limiter la portée.

### EXEMPLE 1

On prépare la composition suivante :

| | |
|---|---|
| Composé (A) | 10⁻³ mole % |
| Eau distillée | qsp 100 |

Le composé (A) a la structure suivante :

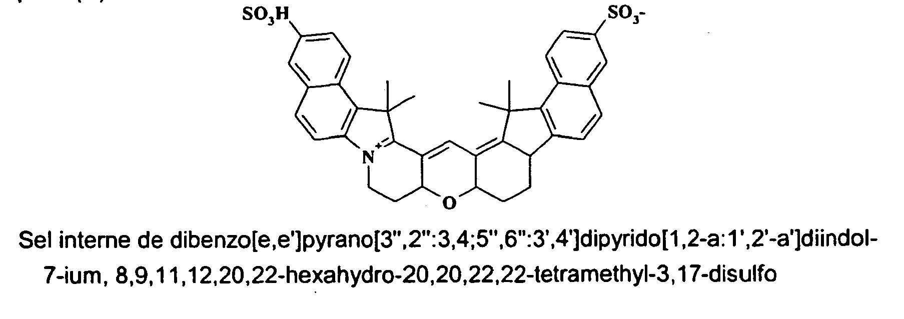

La composition contenant A est appliquée sur cheveux blancs naturels, pendant 20 minutes à température ambiante. Le rapport de bain est fixé à 5. Après teinture, les mèches sont rincées et séchées.

La couleur violette obtenue est tenace aux shampooings.
La composition est stable au stockage.

### EXEMPLE 2

On prépare la composition suivante :

| | |
|---|---|
| Composé (B) | 10⁻³ mole % |
| Eau distillée | qsp 100 |

Le composé (B) a la structure suivante :

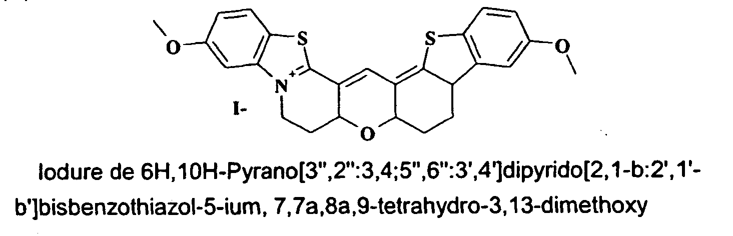

La composition contenant B est appliquée sur cheveux blancs naturels, pendant 20 minutes à température ambiante. Le rapport de bain est fixé à 5. Après teinture, les mèches sont rincées et séchées.

La couleur bleu obtenue est tenace aux shampooings.
La composition est stable au stockage.

### EXEMPLE 3

**Les compositions de colorations suivantes sont préparées :**

| | |
|---|---|
| Colorant direct méthinique de formule A, B ou C | 10⁻³ mol% |
| Hydroxyéthylcellulose vendue par la société Aqualon sous la dénomination Natrosol250MR | 0,384% |
| Melange de p-hydroxybenzoates de méthyle, éthyle, propyle, butyle et isobutyle vendu par la société NIPA sous la dénomination de NIPA ester 82121 | 0,032% |
| Alkyl(C8/C10 50/50)polyglucoside vendu par la société SEPPIC sous la dénomination Oramix CG110 | 5% |
| Alcool benzylique | 4% |
| Propylèneglycol (80E) | 6% |
| Eau déminéralisée qsp | 100 |

Les composés (A) et (B) sont tel que définis précédemment

La composition colorante contenant A est appliquée sur cheveux à 90% de blancs naturels. Le rapport de bain, la température et le temps de pause sont respectivement 1, 33°C et 30 minutes. Après rinçage, shampooing et séchage au casque pendant 30 minutes, les cheveux présentent une coloration violette très esthétique.

La couleur obtenue est tenace aux shampooings.
La composition est stable au stockage.

La composition colorante contenant B est appliquée sur cheveux à 90% de blancs naturels. Le rapport de bain, la température et le temps de pause sont respectivement 1, 33°C et 30 minutes. Après rinçage, shampooing et séchage au casque pendant 30 minute.. La couleur obtenue rouge est tenace aux shampooings.
La composition est stable au stockage.

Le composé (C) a la structure suivante :

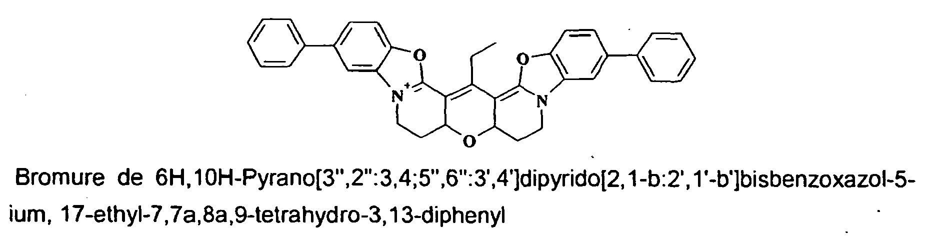

La composition contenant C est appliquée sur cheveux à 90% de blancs naturels.
Le rapport de bain, la température et le temps de pause sont respectivement 1, 33°C et 30 minutes. Après rinçage, shampooing et séchage au casque pendant 30 minute.. La couleur obtenue orange est tenace aux shampooings. La composition est stable au stockage.

## Revendications

1. Composition de coloration de fibres kératiniques humaines comprenant, dans un milieu cosmétiquement acceptable comprenant de l'eau ou un mélange d'eau et de solvant organique, au moins un agent tensioactif et/ou au moins un polymer épaississant, et au moins un colorant direct soluble dans ledit milieu, de formules (I) ou (I') suivantes: 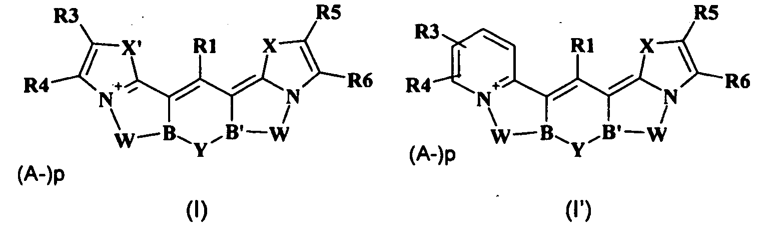 dans lesquelles :
R₁ représente un atome d'hydrogène, un radical alkyle linéaire, ramifié, cyclique ou comprenant 1 à 22 atomes de carbone, plus particulièrement 1 à 6 atomes de carbone, éventuellement substitué par au moins un groupement hydroxyle, par au moins un groupement alcoxy en C₁-C₆ linéaire ou ramifié, par au moins un groupement cycloalcoxy en C₁-C₆, par au moins un groupement phényle lui-même éventuellement substitué par au moins un groupement carboxy, un radical aryle en C₈-C₃₀, un radical amino substitué par un ou plusieurs radicaux identiques ou non, alkyle ou hydroxyalkyle en C₁-C₆, linéaires ou ramifiés R₃, R₄, R₅ et R₆ indépendamment l'un de l'autre, représentent un atome d'hydrogène ; un radical alkyle, linéaire ou ramifié, comprenant 1 à 22 atomes de carbone, plus particulièrement 1 à 10 atomes, de préférence de 1 à 6 atomes de carbone, éventuellement substitué par un ou plusieurs radicaux hydroxyle ; un atome d'halogène, un radical carboxy, un radical sulfo ;
R₃ et R₄ et/ou R₅ et R₆ peuvent former deux à deux avec les atomes de carbone auquel chacun est rattaché, un cycle ou un hétérocycle aliphatique ou aromatique comprenant de 6 à 30 chaînons, éventuellement condensé à un cycle ou hétérocycle aromatique identique ou non comprenant de 6 à 30 chaînons ; les cycles ou hétérocycles aliphatiques ou aromatiques étant éventuellement substitués par au moins un atome d'halogène ; par au moins un radical alcoxy en C₁-C₆, par au moins un radical carboxy, par au moins un radical sulfo et/ou par au moins un radical aryle en C₆-C₃₀ par au moins un radical alkyle linéaire ou ramifié en C₁-C₆, ledit radical alkyle étant éventuellement interrompu par un groupement aminocarbonyle ou carbonylamino et éventuellement terminé par un groupement hydroxyle, carboxy, amino, hydrogénocarbonylamino.
B, B' indépendamment l'un de l'autre représentent un atome d'azote ou un groupement CH ;
W représente un radical divalent comportant deux atomes de carbone, de telle sorte que l'enchaînement N-W-B comporte ou non une insaturation, ledit radical divalent étant éventuellement substitué par une chaîne alkyle en C₁-C₆, aryl(C₆-C₃₀)oxy alkyle(C₁-C₆), ou alkyl(C₁-C₄)arylC₆-C₃₀)amino ;
X, X' indépendamment l'un de l'autre représentent O, S, N, NR'₇, Se, CR'₈R'₉ ;
Y représente O, S, N, Se, NR₇, CO, CR₈R₉ ;
R₈, R₉ indépendamment l'un de l'autre, représentent un atome d'hydrogène ; un radical alkyle linéaire ou ramifié en C₁-C₂₂ éventuellement substitué par un ou plusieurs radicaux hydroxyle, mono ou dialkylamino en C₁-C₁₀, mono ou dihydroxylalkylamino en C₁-C₁₀, aryle en C₁₀-C₃₀, aryloxy en C₁₀-C₃₀, acyl(C₂-C₁₀)amino ;
R₇ représente un atome d'hydrogène, un radical alkyle linéaire ou ramifié en C1-C10, un radical aryle en C6-C30, un radical amino porteur d'au moins un radical aryle en C₆-C₃₀ éventuellement substitué par au moins un radical alkyle en C₁-C₈ ou au moins un radical carboxy, d'au moins un radical alkyl(C₁-C₄)aryl(C₆-C₃₀)sulfonyle, ou au moins un radical acyle en C₂-C₁₀ ou au moins un radical trialkyl(C₁-C₄)aminoalkyl(C₁-C₄)carbonyle, ou au moins un groupement aminothiocarbonyle ;
R₇ peut aussi représenter un radical Z ou -N=Z, Z désignant un hétérocycle à 5 ou 6 chaînons éventuellement condensé et comportant de 1 à 30 atomes de carbone, un ou plusieurs des atomes de carbone pouvant être remplacé par un groupement CO ; R'₇ représente un atome d'hydrogène, un radical alkyle en C₁-C₆, un radical hydroxyalkyle en C₁-C₆ ;
R'₈ et R'₉ indépendamment l'un de l'autre représentent un atome d'hydrogène, un radical alkyle en C₁-C₆, un radical hydroxyalkyle en C₁-C₆, un radical carboxyalkyle en C₁-C₆, un radical alcoxyC₁-C₄carbonylyalkyle en C₁-C₆ ;
p est un entier égal à 0 ou 1 ;
A⁻ représente un anion organique ou minéral, ou un mélange d'anions.

2. Composition selon la revendication précédente, **caractérisée en ce que** le radical R₁ est un atome d'hydrogène, un radical alkyle linéaire ou ramifié en C₁-C₁₆ non substitué.

3. Composition selon l'une quelconque des revendications précédentes, **caractérisée en ce que** Y représente O, N, NR₇, CO.

4. Composition selon l'une quelconque des revendications précédentes, **caractérisée en ce que** X et X' désignent indépendamment l'un de l'autre un atome de soufre, un atome d'oxygène, un groupement NR'₇ avec R représentant un radical alkyle en C₁-C₄.

5. Composition selon l'une quelconque des revendications précédentes, **caractérisée en ce que** X et X' sont identiques.

6. Composition selon l'une quelconque des revendications précédentes, **caractérisée en ce que** W désigne un radical éthylène.

7. Composition selon l'une quelconque des revendications précédentes, **caractérisée en ce que** B et B' désignent un atome de carbone ou un groupement CH.

8. Composition selon l'une quelconque des revendications précédentes, **caractérisée en ce que** R₃ et R₄ et/ou R₅ et R₆ forment deux à deux avec les atomes de carbone auquel chacun est rattaché, un cycle ou un hétérocycle aliphatique ou aromatique comprenant de 6 à 30 chaînons condensé à un cycle ou hétérocycle aromatique identique ou non comprenant de 6 à 30 chaînons ; les cycles ou hétérocycles aliphatiques ou aromatiques étant éventuellement substitués par au moins un atome d'halogène ; par au moins un radical alcoxy en C₁-C₆, par au moins un radical carboxy, par au moins un radical sulfo et/ou par au moins un radical aryle en C₆-C₃₀ par au moins un radical alkyle linéaire ou ramifié en C₁-C₆, ledit radical alkyle étant éventuellement interrompu par un groupement aminocarbonyle ou carbonylamino et éventuellement terminé par un groupement hydroxyle, carboxy, amino, hydrogénocarbonylamino.

9. Composition selon l'une quelconque des revendications précédentes, **caractérisée en ce que** l'anion d'origine minérale est choisi parmi les halogénures, les sulfates, les bisulfates, les nitrates, les phosphates, les hydrogénophosphates, les dihydrogénophosphates, les carbonate, les bicarbonates ; et **en ce que** l'anion d'origine organique, est choisi parmi ceux provenant de sels d'acides mono- ou polycarboxyliques, sulfoniques, sulfuriques, saturés ou non, aromatiques ou non, éventuellement substitués par au moins un radical hydroxyle, amino, ou atomes d'halogène.

10. Composition selon la revendication précédente, **caractérisée en ce que** l'anion est choisi parmi le chlorure, l'iodure, le sulfate, le méthosulfate, l'éthosulfate.

11. Composition selon l'une quelconque des revendications précédentes, **caractérisée en ce que** le colorant direct correspond à l'une des composés suivants :
• Iodure de Benzimidazo[2,1-f]benzimidazo[1',2':1,2]pyrido[4,3-b][1,6] naphthyridinium, 2,3,13,14-tetrachloro-16,18-diethyl-6,7,7a,8,8a,9,10,16-octahydro -8-[(4-methylphenyl)amino]
• Iodure de 6H-Benzothiazolo[2,3-f]benzothiazolo[3',2':1,2]pyrido[4,3-b][1,6] naphthyridin-5-ium, 8-(2-benzothiazolylamino)-7,7a,8,8a,9,10-hexahydro
• Chlorure iodure de 6H-Benzothiazolo[2,3-f]benzothiazolo[3',2':1,2]pyrido[4,3-b][1,6]naphthyridin-5-ium, 7,7a,8,8a,9,10-hexahydro-8-[[(trimethylammonio) acetyl]amino]-,
• Iodure de 6H-Benzothiazolo[2,3-f]benzothiazolo[3',2':1,2]pyrido[4,3-b][1,6] naphthyridin-5-ium, 7,7a,8,8a,9,10-hexahydro-8-(4-oxo-2-thioxo-3-thiazolidinyl)
• Iodure de 6H-Benzothiazolo[2,3-f]benzothiazolo[3',2':1,2]pyrido[4,3-b][1,6] naphthyridin-5-ium, 7,7a,8,8a,9,10-hexahydro-8-[[(4-methylphenyl)sulfonyl]amino]
• Iodure de 6H-Benzothiazolo[2,3-f]benzothiazolo[3',2':1,2]pyrido[4,3-b][1,6] naphthyridin-5-ium, 8-[(1,1-dioxido-1,2-benzisothiazol-3-yl)amino]-7,7a,8,8a,9,10-hexahydro
• Trifluoroacetate de6H-Pyrano[3",2":3,4;5",6":3',4']dipyrido[1,2-a:1';2'-a']bis benzimidazolium, 2,3,13,14-tetrachloro-16,18-diethyl-7,7a,8a,9,10,16-hexahydro
• Bromure de 6H,10H-Pyrano[3",2":3,4;5",6":3',4']dipyrido[2,1-b:2',1'-b']bis benzoxazol-5-ium, 17-ethyl-7,7a,8a,9-tetrahydro-3,13-diphenyl
• Iodure de 6H,10H-Pyrano[3",2":3,4;5",6":3',4']dipyrido[2,1-b:2',1'-b']bis benzothiazol-5-ium, 7,7a,8a,9-tetrahydro-3,13-dimethoxy
• Bromure de 6H,10H-Pyrano[3",2":3,4;5",6":3',4']dipyrido[2,1-b:2',1'-b']bis benzothiazol-5-ium, 17-ethyl-7,7a,8a,9-tetrahydro
• Iodure de 6H,10H-Pyrano[3",2":3,4;5",6":3',4']dipyrido[2,1-b:2',1'-b']bisbenzo thiazol-5-ium, 7,7a,8a,9-tetrahydro
• Bromure de 6H-Benzoxazolo[2,3-f]benzoxazolo[3',2':1,2]pyrido[4,3-b][1,6] naphthyridin-5-ium, 8-(2-benzothiazolylamino)-7,7a,8,8a,9,10-hexahydro-3,13-diphenyl
• Bromure de 6H-Benzothiazolo[2,3-f]benzothiazolo[3',2':1,2]pyrido[4,3-b][1,6] naphthyridin-5-ium, 8-(2-benzothiazolylamino)-7,7a,8,8a,9,10-hexahydro
• Iodure de 6H-Benzothiazolo[2,3-f]benzothiazolo[3',2':1,2]pyrido[4,3-b][1,6] naphthyridin-5-ium, 8-[(aminothioxomethyl)amino]-7,7a,8,8a,9,10-hexahydro
• Iodure de 6H-Benzimidazo[2,1-f]benzimidazo[1',2':1,2]pyrido[4,3-b][1,6] naphthyridin-5-ium, 2,3,13,14-tetrachloro-16,18-diethyl-7,7a,8,8a,9,10,16,18-octahydro-9-[(4-methylphenyl)amino]
• Chlorure de 9H-Bisbenzimidazo[2',1':3,4]pyrazino[1,2-c:2',1'-f]pyrimidin-5-ium, 6,7,11,12,18,20-hexahydro-9-oxo-
• Sel interne de Pyrano[3",2":3,4;5",6":3',4']dipyrido[1,2-a:1',2'-a']diindol-5-ium, 2-(aminomethyl)-6,7,9,10,16,18-hexahydro-16,16,18,18-tetramethyl-14-sulfo-
• Sel interne de Pyrano[3",2":3,4;5",6":3',4']dipyrido[1,2-a:1',2'-a']diindol-5-ium, 2-(carboxymethyl)-6,7,9,10,16,18-hexahydro-16,16,18,18-tetramethyl-14-sulfo
• Sel interne de Pyrano[3",2":3,4;5",6":3',4']dipyrido[1,2-a:1',2'-a']diindol-5-ium, 2,14-bis(carboxymethyl)-6,7,9,10,16,18-hexahydro-16,16,18,18-tetramethyl
• Sel interne de 6H-Benz[2",3"]indolizino[8',7':5,6]pyrano[3,2-a]benzo[f]quinolizin-5-ium, 14-(carboxymethyl)-7,7a,8a,9,10,16-hexahydro-16,16-dimethyl
• Sel interne de 6H-Benz[2,3]indolizino[7,8-b]indolo[2,1-f][1,6]naphthyridin-5-ium, 8-[(4-carboxyphenyl)amino]-7,7a,8,8a,9,10,16,18-octahydro-16,16,18,18-tetramethyl
• Sel interne de 10H-Benz[2",3"]indolizino[8",7":5',6']pyrano[3',2':3,4]pyrido[2,1-b]benzothiazol-5-ium, 2-(carboxymethyl)-6,7,7a,8a,9,18-hexahydro-18,18-dimethyl
• Sel interne de Pyrano[3",2":3,4;5",6":3',4'jdipyrido[1,2-a:1',2'-a']diindol-5-ium, 6,7,9,10,16,18-hexahydro-2-(2-hydroxyethyl)-16,16,18,18-tetramethyl-14-sulfo
• Sel interne de Pyrano[3",2":3,4;5",6":3',4']dipyrido[1,2-a:1',2'-a']diindol-5-ium, 2-[(formylamino)methyl]-6,7,9,10,16,18-hexahydro-16,16,18,18-tetramethyl-14-sulfo
• Sel interne de Pyrano[3",2":3,4;5",6":3',4']dipyrido[1,2-a:1',2'-a']diindol-5-ium, 2-[2-[(2-aminoethyl)amino]-2-oxoethyl]-6,7,9,10,16,18-hexahydro-16,16,18,18-tetramethyl-14-sulfo
• Sel interne de Pyrano[3",2":3,4;5",6":3',4']dipyrido[1,2-a:1',2'-a']diindol-5-ium, 2-[2-[(2-carboxymethyl)amino]-2-oxoethyl]-6,7,9,10,16,18-hexahydro-,16,16,18,18-tetramethyl-14-sulfo
• Sel interne de Dibenzo[e,e']pyrano[3",2":3,4;5",6":3',4']dipyrido[1,2-a:1',2'-a']diindol-7-ium, 8,9,11,12,20,22-hexahydro-20,20,22,22-tetramethyl-3,17-disulfo
• Sel (par exemple chlorure) de 7H,11H-Bisnaphth[2',3':4,5]oxazolo[3,2-a:3',2'-a'] pyrano[3,2-c:5,6-c']dipyridin-6-ium
• 4-methylbenzenesulfonate de 7H,11H-Bisnaphth[2',3':4,5]oxazolo[3,2-a:3',2'-a'] pyrano [3,2-c:5,6-c']dipyridin-6-ium, 20-ethyl-8,8a,9a,10-tetrahydro-8,10-bis(3-phenoxypropyl)-,
• Sel (par exemple chlorure) de 7H,11H-Bisnaphth[2',3':4,5]oxazolo[3,2-a:3',2'-a']pyrano[3,2-c:5,6-c']dipyridin-6-ium, 8,8a,9a,10-tetrahydro-8,10-bis(3-phenoxy propyl)-
• Sel (par exemple chlorure) de 6H,10H-Naphtho[1"",2"":4"',5"'] thiazolo[3'",2"':1",2"]pyrido [3",4":5',6']pyrano[3',2':3,4 ]pyrido[2,1-b]benzoxazol-5-
• Bromure de 6H,10H-Pyrano[3",2":3,4;5",6":3',4']dipyrido[2,1-b:2',1'-b']bis benzothiazol-5-ium, 7,7a,8a,9-tetrahydro
• Sel (par exemple chlorure) de 10H-Benz[2",3"]indolizino[8",7":5',6']pyrano [3',2':3,4]pyrido [2,1-b]benzothiazol-5-ium
• Sel (par exemple chlorure) de Dibenzo[e,e']pyrano[3",2":3,4;5",6":3',4']dipyrido[1,2-a:1',2'-a']diindol-7-
• Sel (par exemple chlorure) de Pyrano[3",2":3,4;5",6":3',4']dipyrido[1,2-a:1',2'-a'] diindol-5-ium
• Sel (par exemple chlorure) de 1H-Benz[2,3]indolizino[7,8-b]indolo[2,1-f][1,6] naphthyridin-5-ium
• Heptafluorobutanoate 6H,10H-Pyrano[3",2":3,4;5",6":3',4']dipyrido[2,1-b:2',1'-b']bis benzothiazol-5-ium, 7,7a,8a,9-tetrahydro-,
• Sulfate de 6H-Pyrano[3",2":3,4;5",6":3',4']dipyrido[1,2-a:1',2'-a']bis benzimidazolium, 2,3,13,14-tetrachloro-16,18-diethyl-7,7a,8a,9,10,16-hexahydro
• Sel (par exemple chlorure) de 6H-Benzothiazolo[2,3-f]benzo thiazolo [3',2':1,2]pyrido[4,3-b][1,6]naphthyridin-5-ium,8-(acetylamino)-7,7a,8,8a,9, 10-hexahydro
• Sel (par exemple chlorure) de 1H-Benzimidazo[2,1-f]benzimidazo [1',2':1,2]pyrido[4,3-b][1,6]naphthyridin-5-ium
• Iodure de Benzimidazo[2,1-f]benzimidazo[1',2':1,2]pyrido[4,3-b][1,6] naphthyridinium, 2,3,13,14-tetrachloro-16,18-diethyl-6,7,7a,8,8a,9,10,16-octahydro -8-[(4-methylphenyl)amino]-,
• Sel (par exemple chlorure) de Pyrano[3",2":3,4;5",6":3',4']dipyrido[1,2-a:1',2'-a']bis benzimidazol-5-
• Sel (par exemple chlorure) de 6H,10H-Pyrano[3",2":3,4;5",6":3',4']dipyrido[2,1-b:2',1 '-b']bisbenzothiazol-5-ium
• Sel (par exemple chlorure) de 6H-Benzothiazolo[2,3-f]benzo thiazolo [3',2': 1,2]pyrido[4,3-b][1,6]naphthyridin-5-ium
• Sel (par exemple chlorure) de 6H,10H-Pyrano[3",2":3,4;5",6":3',4']dipyrido[2,1-b:2',1'-b']bisbenzoxazol-5-
• Sel (par exemple chlorure) de 6H-Benzoxazolo[2,3-f]benzoxazolo [3',2':1,2]pyrido[4,3-b][1,6]naphthyridin-5-ium
• Sel (par exemple chlorure) de 6H-Benzimidazo[2,1-f]benzimidazo [1',2': 1,2]pyrido[4,3-b][1,6]naphthyridin-5-ium
• Dichloroiodate 6H-Benzothiazolo[2,3-f]benzothiazolo[3',2':1,2]pyrido[4,3-b][1,6] naphthyridin-5-ium, 8-(acetylamino)-7,7a,8,8a,9,10-hexahydro-,
• Chlorure de 6,7,11,12-tetrahydro-9-oxo-9H-bisthiazolo[3,2-a:3',2'-a']-pyrimido[6,1-c:4,3-c']dipyrazin-5-ium
• Bromure de 6,7,11,12-tetrahydro-9-oxo-9H-bisoxazolo[3,2-a:3',2'-a']-pyrimido[6,1-c:4,3-c']dipyrazin-5-ium
• Bromure de 6,7,11,12-tetrahydro-9-oxo-9H-bisselenazolo[3,2-a:3',2'-a']-pyrimido [6,1-c:4,3-c']dipyrazin-5-ium.

12. Composition selon l'une quelconque des revendications précédentes, **caractérisée en ce que** la teneur en colorant(s) direct(s) de formules (I) ou (I') est comprise entre 0,01 et 20% en poids par rapport au poids total de la composition.

13. Composition selon la revendication précédente, **caractérisée en ce que** la teneur en colorant(s) direct(s) de formules (I) ou (I') est comprise entre 0,1 et 5 % en poids, par rapport au poids total de la composition.

14. Composition selon l'une quelconque des revendications précédentes, **caractérisée en ce qu'**elle comprend en outre au moins un colorant direct additionnel de nature non ionique, cationique ou anionique.

15. Composition selon la revendication précédente, **caractérisée par le fait que** les colorants directs additionnels sont choisis parmi les colorants benzéniques nitrés, les colorants azoïques, les colorants anthraquinoniques, naphtoquinoniques ou benzoquinoniques, les colorants indigoïdes, ou les colorants dérivés du triarylméthane, les colorants naturels, ou leurs mélanges.

16. Composition selon l'une quelconque des revendications 14 ou 15, **caractérisée en ce que** le ou les colorants directs additionnels représentent de 0,0005 à 12 % en poids par rapport au poids total de la composition.

17. Composition selon la revendication 1, **caractérisée en ce que** l'agent tensioactif est non ionique.

18. Composition selon l'une des revendications précédentes, **caractérisée en ce que** la teneur en tensioactifs représente de 0,01 et 50 % en poids par rapport au poids total de la composition.

19. Composition selon l'une quelconque des revendications précédentes, **caractérisée en ce qu'**elle contient au moins un polymère épaississant non associatif.

20. Composition selon la revendication précédente, **caractérisée en ce qu'**elle contient au moins un polymère épaississant non associatif choisi les homopolymères d'acide acrylique réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide, les gommes de guar non ioniques, les gommes de biopolysaccharides d'origine microbienne, les gommes issues d'exudats végétaux, les hydroxypropyl- ou carboxyméthyl- celluloses ; les pectines et les alginates, seuls ou en mélanges.

21. Composition selon l'une quelconque des revendications précédentes, **caractérisée en ce** q'elle contient au moins un polymère épaississant associatif.

22. Composition selon la revendication précédente, **caractérisée en ce** q'elle contient au moins un polymère épaississant associatif choisi parmi les polyuréthanes associatifs, plus particulièrement cationiques ou non ioniques, les dérivés de cellulose associatifs, plus particulièrement cationiques ou non ioniques, les vinyllactames associatifs, les polyacides insaturés associatifs, les aminoplaste-éthers associatifs, les homopolymères réticulés d'acide 2-acrylamido-2-méthylpropane sulfonique et leurs copolymères réticulés d'acrylamide, les polymères ou copolymères associatifs comprenant au moins un monomère à insaturation éthylénique à groupement sulfonique, seuls ou en mélanges.

23. Composition selon l'une quelconque des revendications 19 à 22, **caractérisée en ce que** la teneur en polymère épaississant associatif ou non représente 0,01 et 10% en poids, plus particulièrement 0,1 à 5% en poids, par rapport au poids de la composition.

24. Composition selon l'une quelconque des revendications précédentes, **caractérisée en ce qu'**elle se présente sous la forme d'un shampooing colorant.

25. Composition selon l'une quelconque des revendications 1 à 23**, caractérisée en ce qu'**elle comprend au moins une base d'oxydation éventuellement associée à au moins un coupleur.

26. Composition selon la revendication 25, **caractérisée en ce qu'**elle comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

27. Composition selon la revendication précédente, **caractérisée en ce que** la teneur en base(s) d'oxydation représente de 0,0005 à 12 % en poids par rapport au poids total de la composition.

28. Composition selon l'une quelconque des revendications 25 à 27, **caractérisée en ce qu'**elle comprend au moins un coupleur choisi parmi les métaphénylènediamines, les méta-aminophénols, les métadiphénols et les coupleurs hétérocycliques ou leurs sels d'addition avec un acide ou avec un agent alcalin.

29. Composition selon la revendication précédente, **caractérisée en ce que** la teneur en coupleur(s) représente de 0,0001 à 10 % en poids par rapport au poids total de la composition tinctoriale.

30. Composition selon l'une quelconque des revendications précédentes, **caractérisée en ce qu'**elle comprend au moins un adjuvant choisi parmi les polymères anioniques, cationiques, non ioniques, amphotères, zwittérioniques ou leurs mélanges ; les agents épaississants minéraux ; les agents antioxydants ; les agents de pénétration ; les agents séquestrants ; les parfums ; les tampons ; les agents dispersants ; les agents de conditionnement ; les agents filmogènes ; les céramides ; les agents conservateurs ; les agents stabilisants ; les agents opacifiants.

31. Composition selon l'une quelconque des revendications 1 à 23 et 25 à 30,
**caractérisée en ce qu'**elle renferme au moins un agent oxydant.

32. Composition selon la revendication précédente, **caractérisée en ce que** l'agent oxydant est choisi parmi le peroxyde d'hydrogène, le peroxyde d'urée, les bromates de métaux alcalins, les persels tels que les perborates et persulfates, et les enzymes.

33. Procédé de traitement de fibres kératiniques plus particulièrement de fibres kératiniques humaines, **caractérisé en ce que** l'on applique sur lesdites fibres, sèches ou humides, une composition telle que définie selon l'une quelconque des revendications précédentes, pendant un temps suffisant pour développer la coloration, après quoi on rince, on lave éventuellement au shampooing, on rince à nouveau et on sèche ou on laisse sécher les fibres résultantes.

34. Procédé de traitement de fibres kératiniques plus particulièrement de fibres kératiniques humaines, **caractérisé en ce que** l'on applique sur lesdites fibres, sèches ou humides, une composition telle que définie selon l'une quelconque des revendications 1 à 23 sans rinçage final.

35. Dispositif à plusieurs compartiments pour la teinture et l'éclaircissement des cheveux, comprenant au moins un compartiment renfermant une composition selon l'une quelconque des revendications 1 à 23 et 25 à 30, et au moins un autre compartiment renfermant une composition renfermant au moins un agent oxydant.

36. Utilisation de la composition selon l'une quelconque des revendications 1 à 32 comme agent colorant les fibres kératiniques, en particulier des fibres kératiniques humaines.

## Claims

1. Composition for dyeing human keratin fibres comprising, in a cosmetically acceptable medium comprising water or a mixture of water and an organic solvent, at least one surfactant and/or at least one thickening polymer, and at least one direct dye that is soluble in the said medium, of formula (I) or (I') below: 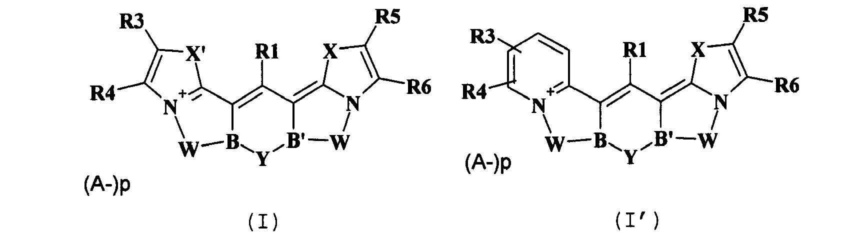 in which:
R1 represents a hydrogen atom, a linear, branched or cyclic alkyl radical containing 1 to 22 carbon atoms and more particularly 1 to 6 carbon atoms, optionally substituted with at least one hydroxyl group, with at least one linear or branched C₁-C₆ alkoxy group, with at least one C₁-C₆ cycloalkoxy group, with at least one phenyl group, itself optionally substituted with at least one carboxyl group, a C₆-C₃₀ aryl radical, an amino radical substituted with one or more linear or branched C₁-C₆ alkyl or hydroxyalkyl radicals, which may be identical or different, R3, R4, R5 and R6, independently of each other, represent a hydrogen atom; a linear or branched alkyl radical containing 1 to 22 carbon atoms, more particularly 1 to 10 carbon atoms and preferably from 1 to 6 carbon atoms, optionally substituted with one or more hydroxyl radicals; a halogen atom, a carboxyl radical, a sulfo radical;
R3 and R4 and/or R5 and R6 may form in pairs, with the carbon atoms to which each is attached, a 6- to 30-membered aliphatic or aromatic ring or heterocycle, optionally fused to an identical or different 6- to 30-membered aromatic ring or heterocycle; the aliphatic or aromatic rings or heterocycles being optionally substituted with at least one halogen atom; with at least one C₁-C₆ alkoxy radical, with at least one carboxyl radical, with at least one sulfo radical and/or with at least one C₆-C₃₀ aryl radical with at least one linear or branched C₁-C₆ alkyl radical, the said alkyl radical being optionally interrupted with an aminocarbonyl or carbonylamino group and optionally ending with a hydroxyl, carboxyl, amino or hydrogenocarbonylamino group,
B and B', independently of each other, represent a nitrogen atom or a CH group;
W represents a divalent radical containing two carbon atoms, such that the sequence N-W-B does or does not comprise an unsaturation, the said divalent radical being optionally substituted with a C₁-C₆ alkyl, (C₆-C₃₀) aryloxy (C₁-C₆) alkyl or (C₁-C₄) alkyl (C₆-C₃₀)-arylamino chain;
X and X', independently of each other, represent O, S, N, NR' 7, Se or CR' 8R' 9;
Y represents O, S, N, Se, NR7, CO or CR8R9;
R8 and R9, independently of each other, represent a hydrogen atom; a linear or branched C₁-C₂₂ alkyl radical optionally substituted with one or more hydroxyl, C₁-C₁₀ mono- or dialkylamino, C₁-C₁₀ mono- or dihydroxyalkylamino, C₁₀-C₃₀ aryl, C₁₀-C₃₀ aryloxy or (C₂-C₁₀) acylamino radicals;
R7 represents a hydrogen atom, a linear or branched C₁-C₁₀ alkyl radical, a C₆-C₃₀ aryl radical, an amino radical bearing at least one C₆-C₃₀ aryl radical optionally substituted with at least one C₁-C₆ alkyl radical or at least one carboxyl radical, at least one (C₁-C₄) alkyl (C₆-C₃₀) arylsulfonyl radical, or at least one C₂-C₁₀ acyl radical or at least one tri(C₁-C₄)-alkylamino(C₁-C₄)alkylcarbonyl radical, or at least one aminothiocarbonyl group;
R7 may also represent a radical Z or -N=Z, Z denoting an optionally fused 5- or 6-membered heterocycle containing from 1 to 30 carbon atoms, one or more of the carbon atoms possibly being replaced with a CO group;
R'7 represents a hydrogen atom, a C₁-C₆ alkyl radical or a C₁-C₆ hydroxyalkyl radical;
R'8 and R'9, independently of each other, represent a hydrogen atom, a C₁-C₆ alkyl radical, a C₁-C₆ hydroxyalkyl radical, a C₁-C₆ carboxyalkyl radical or a (C₁-C₄)alkoxycarbonyl(C₁-C₆)alkyl radical;
p is an integer equal to 0 or 1;
A⁻ represents an organic or mineral anion, or a mixture of anions.

2. Composition according to the preceding claim, **characterized in that** the radical R1 is a hydrogen atom or an unsubstituted linear or branched C₁-C₁₆ alkyl radical.

3. Composition according to either of the preceding claims, **characterized in that** Y represents O, N, NR7 or CO.

4. Composition according to any one of the preceding claims, **characterized in that** X and X' denote, independently of each other, a sulfur atom, an oxygen atom or a group NR'7 with R representing a C₁-C₄ alkyl radical.

5. Composition according to any one of the preceding claims, **characterized in that** X and X' are identical.

6. Composition according to any one of the preceding claims, **characterized in that** W denotes an ethylene radical.

7. Composition according to any one of the preceding claims, **characterized in that** B and B' denote a carbon atom or a CH group.

8. Composition according to any one of the preceding claims, **characterized in that** R3 and R4 and/or R5 and R6 form in pairs, with the carbon atoms to which each is attached, a 6- to 30-membered aliphatic or aromatic ring or heterocycle, fused to an identical or different 6- to 30-membered aromatic ring or heterocycle; the aliphatic or aromatic rings or heterocycles being optionally substituted with at least one halogen atom; with at least one C₁-C₆ alkoxy radical, with at least one carboxyl radical, with at least one sulfo radical and/or with at least one C₆-C₃₀ aryl radical, with at least one linear or branched C₁-C₆ alkyl radical, the said alkyl radical being optionally interrupted with an aminocarbonyl or carbonylamino group and optionally ending with a hydroxyl, carboxyl, amino or hydrogenocarbonylamino group.

9. Composition according to any one of the preceding claims, **characterized in that** the anion of mineral origin is chosen from halides, sulfates, bisulfates, nitrates, phosphates, hydrogen phosphates, dihydrogen phosphates, carbonates and bicarbonates; and **in that** the anion of organic origin is chosen from anions originating from salts of saturated or unsaturated, aromatic or non-aromatic sulfonic, sulfuric, mono- or polycarboxylic acids, optionally substituted with at least one hydroxyl or amino radical or halogen atoms.

10. Composition according to the preceding claim, **characterized in that** the anion is chosen from chloride, iodide, sulfate, methosulfate and ethosulfate.

11. Composition according to any one of the preceding claims, **characterized in that** the direct dye corresponds to one of the following compounds:
• Benzimidazo[2,1-f]benzimidazo[1',2':1,2]pyrido[4,3-b][1,6]naphthyridinium, 2,3,13,14-tetrachloro-16,18-diethyl-6,7,7a,8,8a,9,10,16-octahydro-8-[(4-methylphenyl)amino], iodide
• 6H-Benzothiazolo[2,3-f]benzothiazolo[3',2':1,2]-pyrido[4,3-b][1,6]naphthyridin-5-ium, 8-(2-benzothiazolylamino)-7,7a,8,8a,9,10-hexahydro, iodide
• 6H-Benzothiazolo[2,3-f]benzothiazolo[3',2':1,2]-pyrido[4,3-b][1,6]naphthyridin-5-ium, 7,7a,8,8a,9,10-hexahydro-8-[[(trimethylammonio)acetyl]amino]-, chloride iodide
• 6H-Benzothiazolo[2,3-f]benzothiazolo[3',2':1,2]-pyrido[4,3-b][1,6]naphthyridin-5-ium, 7,7a,8,8a,9,10-hexahydro-8-(4-oxo-2-thioxo-3-thiazolidinyl), iodide
• 6H-Benzothiazolo[2,3-f]benzothiazolo[3',2':1,2]-pyrido[4,3-b][1,6]naphthyridin-5-ium, 7,7a,8,8a,9,10-hexahydro-8-[[(4-methylphenyl)sulfonyl]amino], iodide
• 6H-Benzothiazolo[2,3-f]benzothiazolo[3',2':1,2]-pyrido[4,3-b][1,6]naphthyridin-5-ium, 8-[(1,1-dioxido-1,2-benzisothiazol-3-yl)amino]-7,7a,8,8a,9,10-hexahydro, iodide
• 6H-Pyrano[3",2":3,4;5",6":3',4']dipyrido[1,2-a:1',2'-a']bisbenzimidazolium, 2,3,13,14-tetrachloro-16,18-diethyl-7,7a,8a,9,10,16-hexahydro, trifluoroacetate
• 6H, 10H-Pyrano [3",2":3,4;5", 6":3',4']dipyrido[2,1-b:2',1'-b']bisbenzoxazol-5-ium, 17-ethyl-7,7a,8a,9-tetrahydro-3,13-diphenyl, bromide
• 6H, 10H-Pyrano [3",2":3,4;5", 6":3',4']dipyrido[2,1-b:2',1'-b']bisbenzothiazol-5-ium, 7,7a,8a,9-tetrahydro-3,13-dimethoxy, iodide
• 6H, 10H-Pyrano[3",2":3,4;5",6":3',4']dipyrido[2, 1-b:2',1'-b']bisbenzothiazol-5-ium, 17-ethyl-7,7a,8a,9-tetrahydro, bromide
• 6H,10H-Pyrano [3",2":3,4;5",6":3',4']dipyrido[2,1-b:2',1'-b']bisbenzothiazol-5-ium, 7,7a,8a,9-tetrahydro, iodide
• 6H-Benzoxazolo[2,3-f]benzoxazolo[3',2':1,2]pyrido-[4,3-b][1,6]naphthyridin-5-ium, 8-(2-benzothiazolylamino)-7,7a,8,8a,9,10-hexahydro-3,13-diphenyl, bromide
• 6H-Benzothiazolo[2,3-f]benzothiazolo[3',2':1,2]-pyrido[4,3-b][1,6]naphthyridin-5-ium, 8-(2-benzothiazolylamino)-7,7a,8,8a,9,10-hexahydro, bromide
• 6H-Benzothiazolo[2,3-f]benzothiazolo[3',2':1,2]-pyrido[4,3-b][1,6]naphthyridin-5-ium, 8-[(aminothioxomethyl)amino]-7,7a,8,8a,9,10-hexahydro, iodide
• 6H-Benzimidazo[2,1-f]benzimidazo[1',2':1,2]pyrido-[4,3-b][1,6]naphthyridin-5-ium, 2,3,13,14-tetrachloro-16,18-diethyl-7,7a,8,8a,9,10,16,18-octahydro-9-[(4-methylphenyl)amino], iodide
• 9H-Bisbenzimidazo[2',1':3,4]pyrazino[1,2-c:2',1'-f]pyrimidin-5-ium, 6,7,11,12,18,20-hexahydro-9-oxo-, chloride
• Internal salt of pyrano [3",2":3,4; 5", 6": 3',4']dipyrido[1,2-a:1',2'-a']diindol-5-ium, 2-(aminomethyl)-6,7,9,10,16,18-hexahydro-16,16,18,18-tetramethyl-14-sulfo-
• Internal salt of pyrano[3",2":3,4;5",6":3',4']dipyrido[1,2-a:1',2'-a']diindol-5-ium, 2-(carboxymethyl)-6,7,9,10,16,18-hexahydro-16,16,18,18-tetramethyl-14-sulfo
• Internal salt of pyrano [3",2":3,4; 5",6":3',4']dipyrido[1,2-a:1',2'-a']diindol-5-ium, 2,14-bis(carboxymethyl)-6,7,9,10,16,18-hexahydro-16,16,18,18-tetramethyl
• Internal salt of 6H-benz [2",3"]indolizino-[8',7':5,6]pyrano[3,2-a]benzo[f]quinolizin-5-ium, 14-(carboxymethyl)-7,7a,8a,9,10,16-hexahydro-16,16-dimethyl
• Internal salt of 6H-benz[2,3]indolizino[7,8-b]-indolo[2,1-f][1,6]naphthyridin-5-ium, 8-[(4-carboxyphenyl)amino]-7,7a,8,8a,9,10,16,18-octahydro-16,16,18,18-tetramethyl
• Internal salt of 10H-benz[2",3"]indolizino-[8",7" : 5',6'] pyrano[3',2':3,4] pyrido[2,1-b] benzothiazol-5-ium, 2-(carboxymethyl)-6,7,7a,8a,9,18-hexahydro-18,18-dimethyl
• Internal salt of pyrano [3",2":3,4;5",6":3',4']-dipyrido[1,2-a:1',2'-a']diindol-5-ium, 6,7,9,10,16,18-hexahydro-2-(2-hydroxyethyl)-16,16,18,18-tetramethyl-14-sulfo
• Internal salt of pyrano [3",2":3,4;5",6":3',4']-dipyrido[1,2-a:1',2'-a']diindol-5-ium, 2-[(formylamino)methyl]-6,7,9,10,16,18-hexahydro-16,16,18,18-tetramethyl-14-sulfo
• Internal salt of pyrano[3",2":3,4;5",6":3',4']-dipyrido[1,2-a:1',2'-a']diindol-5-ium, 2-[2-[(2-aminoethyl)amino]-2-oxoethyl]-6,7,9,10,16,18-hexahydro-16,16,18,18-tetramethyl-14-sulfo
• Internal salt of pyrano [3",2":3,4;5",6":3',4']-dipyrido[1,2-a:l',2'-a']diindol-5-ium, 2-[2-[(2-carboxymethyl)amino]-2-oxoethyl]-6,7,9,10,16,18-hexahydro-,16,16,18,18-tetramethyl-14-sulfo
• Internal salt of dibenzo [e, e'] pyrano [3",2":3,4;5",6":3',4']-dipyrido[1,2-a:1',2'-a']diindol-7-ium, 8,9,11,12,20,22-hexahydro-20,20,22,22-tetramethyl-3,17-disulfo
• Salt (e.g. chloride) of 7H,11H-bisnaphth[2',3':4,5]-oxazolo[3,2-a:3',2'-a'] pyrano[3,2-c:5,6-c']dipyridin-6-ium
• 7H,11H-Bisnaphth[2',3':4,5]oxazolo[3,2-a:3',2'-a']pyrano[3,2-c:5,6-c']dipyridin-6-ium, 20-ethyl-8,8a,9a,10-tetrahydro-8,10-bis(3-phenoxypropyl)-, 4-methylbenzenesulfonate
• Salt (e.g. chloride) of 7H,11H-bisnaphth[2',3':4,5]-oxazolo[3,2-a:3',2'-a']pyrano[3,2-c:5,6-c']dipyridin-6-ium, 8,8a,9a,10-tetrahydro-8,10-bis(3-phenoxypropyl)-
• Salt (e.g. chloride) of 6H,10H-naphtho[1"",2"":-4"',5'"]thiazolo[3'",2"':1",2"]pyrido[3",4":-5',6']pyrano[3',2':3,4]pyrido[2,1-b]benzoxazol-5-
• 6H, 10H-Pyrano [3",2":3,4; 5",6":3',4']dipyrido[2,1-b:2',1'-b']bisbenzothiazol-5-ium, 7,7a,8a,9-tetrahydro, bromide
• Salt (e.g. chloride) of 10H-benz[2",3"]indolizino-[8",7":5',6'] pyrano [3',2':3,4]pyrido[2,1-b]benzothiazol-5-ium
• Salt (e.g. chloride) of dibenzo [e,e'] pyrano [3",2": 3,4;5",6":3',4']dipyrido[1,2-a:1',2'-a']diindol-7-
• Salt (e.g. chloride) of pyrano [3",2":3,4;5",6": 3',4']dipyrido[1,2-a:1',2'-a'] diindol-5-ium
• Salt (e.g. chloride) of 1H-benz[2,3]indolizino[7,8-b]indolo[2,1-f][1,6]naphthyridin-5-ium
• 6H, 10H-Pyrano [3",2":3,4;5",6":3',4']dipyrido[2, 1-b:2',1'-b']bisbenzothiazol-5-ium, 7,7a,8a,9-tetrahydro-, heptafluorobutanoate
• 6H-Pyrano [3",2":3,4;5",6": 3',4' dipyrido [1,2-a:1',2'-a']bisbenzimidazolium, 2,3,13,14-tetrachloro-16,18-diethyl-7,7a,8a,9,10,16-hexahydro, sulfate
• Salt (e.g. chloride) of 6H-benzothiazolo[2,3-f]benzothiazolo[3',2':1,2]pyrido[4,3-b][1,6]naphthyridin-5-ium, 8-(acetylamino)-7,7a,8,8a,9, 10-hexahydro
• Salt (e.g. chloride) of 1H-benzimidazo[2,1-f]benzimidazo[1',2':1,2]pyrido[4,3-b][1,6]naphthyridin-5-ium
• Benzimidazo[2,1-f]benzimidazo[1',2':1,2]pyrido[4,3-b][1,6]naphthyridinium, 2,3,13,14-tetrachloro-16,18-diethyl-6,7,7a,8,8a,9,10,16-octahydro-8-[(4-methylphenyl)amino]-, iodide
• Salt (e.g. chloride) of pyrano [3",2":3,4;5",6": 3',4']dipyrido[1,2-a:1',2'-a']bisbenzimidazol-5-
• Salt (e.g. chloride) of 6H,10H-pyrano[3" ,2" :3,4; 5" ,6" :3',4']dipyrido[2,1-b:2',1'-b']bisbenzothiazol-5-ium
• Salt (e.g. chloride) of 6H-benzothiazolo[2,3-f]benzothiazolo[3',2':1,2]pyrido[4,3-b][1,6]naphthyridin-5-ium
• Salt (e.g. chloride) of 6H,10H-pyrano [3",2":3,4; 5",6":3',4']dipyrido [2,1-b:2',1'-b']bisbenzoxazol-5-
• Salt (e.g. chloride) of 6H-benzoxazolo[2,3-f]benzoxazolo[3',2':1,2]pyrido[4,3-b][1,6]naphthyridin-5-ium
• Salt (e.g. chloride) of 6H-benzimidazo[2,1-f]benzimidazo[1',2':1,2]pyrido[4,3-b][1,6]naphthyridin-5-ium
• 6H-Benzothiazolo[2,3-f]benzothiazolo[3',2':1,2]-pyrido[4,3-b][1,6]naphthyridin-5-ium, 8-(acetylamino)-7,7a,8,8a,9,10-hexahydro-, dichloroiodate
• 6,7,11,12-Tetrahydro-9-oxo-9H-bisthiazolo[3,2-a:3',2'-a']pyrimido[6,1-c:4,3-c']dipyrazin-5-ium chloride
• 6,7,11,12-Tetrahydro-9-oxo-9H-bisoxazolo[3,2-a:3',2'-a']pyrimido[6,1-c:4,3-c']dipyrazin-5-ium bromide
• 6,7,11,12-Tetrahydro-9-oxo-9H-bisselenazolo[3,2-a:3',2'-a']pyrimido[6,1-c:4,3-c']dipyrazin-5-ium bromide.

12. Composition according to any one of the preceding claims, **characterized in that** the content of direct dye(s) of formula (I) or (I') is between 0.01% and 20% by weight relative to the total weight of the composition.

13. Composition according to the preceding claim, **characterized in that** the content of direct dye(s) of formula (I) or (I') is between 0.1% and 5% by weight relative to the total weight of the composition.

14. Composition according to any one of the preceding claims, **characterized in that** it also comprises at least one additional direct dye of non-ionic, cationic or anionic nature.

15. Composition according to the preceding claim, **characterized in that** the additional direct dyes are chosen from nitrobenzene dyes, azo, anthraquinone, naphthoquinone or benzoquinone dyes, indigoid dyes or triarylmethane-based dyes and natural dyes, or mixtures thereof.

16. Composition according to either of Claims 14 and 15, **characterized in that** the additional direct dye(s) represent(s) from 0.0005% to 12% by weight relative to the total weight of the composition.

17. Composition according to Claim 1,
**characterized in that** the surfactant is non-ionic.

18. Composition according to one of the preceding claims, **characterized in that** the content of surfactants represents from 0.01% to 50% by weight relative to the total weight of the composition.

19. Composition according to any one of the preceding claims, **characterized in that** it contains at least one non-associative thickening polymer.

20. Composition according to the preceding claim, **characterized in that** it contains at least one non-associative thickening polymer chosen from crosslinked acrylic acid homopolymers, crosslinked 2-acrylamido-2-methylpropanesulfonic acid homopolymers and the crosslinked acrylamide copolymers thereof, ammonium acrylate homopolymers or copolymers of ammonium acrylate and of acrylamide, nonionic guar gums, biopolysaccharide gums of microbial origin, gums originating from plant exudates, hydroxypropyl- or carboxymethyl celluloses; pectins and alginates, alone or as mixtures.

21. Composition according to any one of the preceding claims, **characterized in that** it contains at least one associative thickening polymer.

22. Composition according to the preceding claim, **characterized in that** it contains at least one associative thickening polymer chosen from associative polyurethanes, which are more particularly cationic or nonionic, associative cellulose derivatives, which are more particularly cationic or nonionic, associative vinyllactams, associative unsaturated polyacids, associative aminoplast-ethers, crosslinked 2-acrylamido-2-methylpropanesulfonic acid homopolymers and crosslinked acrylamide copolymers thereof, associative polymers or copolymers comprising at least one ethylenically unsaturated monomer containing a sulfonic group, alone or as mixtures.

23. Composition according to any one of Claims 19 to 22, **characterized in that** the content of associative or non-associative thickening polymer represents from 0.01% to 10% by weight and more particularly 0.1% to 5% by weight relative to the weight of the composition.

24. Composition according to any one of the preceding claims, **characterized in that** it is in the form of a colouring shampoo.

25. Composition according to any one of Claims 1 to 23, **characterized in that** it comprises at least one oxidation base optionally combined with at least one coupler.

26. Composition according to Claim 25, **characterized in that** it comprises at least one oxidation base chosen from para-phenylenediamines, bis-phenylalkylenediamines, para-aminophenols, ortho-aminophenols and heterocyclic bases, or the addition salts thereof with an acid or with an alkaline agent.

27. Composition according to the preceding claim, **characterized in that** the content of oxidation base(s) represents from 0.0005% to 12% by weight relative to the total weight of the composition.

28. Composition according to any one of Claims 25 to 27, **characterized in that** it comprises at least one coupler chosen from meta-phenylenediamines, meta-aminophenols, meta-diphenols and heterocyclic couplers, or the addition salts thereof with an acid or with an alkaline agent.

29. Composition according to the preceding claim, **characterized in that** the content of coupler(s) represents from 0.0001% to 10% by weight relative to the total weight of the dye composition.

30. Composition according to any one of the preceding claims, **characterized in that** it comprises at least one adjuvant chosen from anionic, cationic, nonionic, amphoteric and zwitterionic polymers or mixtures thereof; mineral thickeners; antioxidants; penetrating agents; sequestering agents; fragrances; buffers; dispersants; conditioning agents; film-forming agents; ceramides; preserving agents; stabilizers; opacifiers.

31. Composition according to any one of Claims 1 to 23 and 25 to 30, **characterized in that** it contains at least one oxidizing agent.

32. Composition according to the preceding claim, **characterized in that** the oxidizing agent is chosen from hydrogen peroxide, urea peroxide, alkali metal bromates, persalts such as perborates and persulfates, and enzymes.

33. Process for treating keratin fibres, more particularly human keratin fibres, **characterized in that** a composition as defined according to any one of the preceding claims is applied to the said wet or dry fibres, for a time that is sufficient to develop the coloration, after which the fibres are rinsed, optionally washed with shampoo, rinsed again and dried, or the resulting fibres are left to dry.

34. Process for treating keratin fibres, more particularly human keratin fibres, **characterized in that** a composition as defined according to any one of Claims 1 to 23 is applied to the said wet or dry fibres without final rinsing.

35. Multi-compartment device for dyeing and lightening the hair, comprising at least one compartment containing a composition according to any one of Claims 1 to 23 and 25 to 30, and at least one other compartment containing a composition containing at least one oxidizing agent.

36. Use of the composition according to any one of Claims 1 to 32 as an agent for dyeing keratin fibres, in particular human keratin fibres.

## Patentansprüche

1. Zusammensetzung zum Färben von menschlichen Keratinfasern, die in einem kosmetisch akzeptablen Medium, das Wasser oder ein Gemisch von Wasser und einem organischen Lösemittel enthält, mindestens eine grenzflächenaktiven Stoff und/oder mindestens ein verdickendes Polymer und mindestens einen in dem Medium löslichen Direktfarbstoff der folgenden Formeln (I) oder (I') enthält: 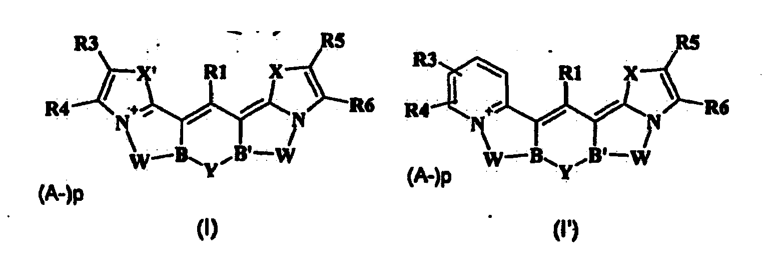 in den Formeln:
R₁ bedeutet ein Wasserstoffatom, eine lineare, verzweigte oder cyclische Alkylgruppe, die 1 bis 22 Kohlenstoffatome und insbesondere 1 bis 6 Kohlenstoffatome enthält und gegebenenfalls mit mindestens einer Hydroxygruppe, mit mindestens einer geradkettigen oder verzweigten C₁₋₆-Alkoxygruppe, mit mindestens einer C₁₋₆-Cycloalkoxygruppe, mit mindestens einer Phenylgruppe substituiert ist, die selbst gegebenenfalls mit mindestens einer Carboxygruppe substituiert ist, eine C₆₋₃₀-Arylgruppe, eine Aminogruppe, die mit einer oder mehreren, gleichen oder voneinander verschiedenen, geradkettigen oder verzweigten Alkyl- oder Hydroxyalkylgruppen mit 1 bis 6 Kohlenstoffatomen substituiert ist; R₃, R₄, R₅ und R₆ bedeuten unabhängig voneinander ein Wasserstoffatom; eine geradkettige oder verzweigte Alkylgruppe mit 1 bis 22 Kohlenstoffatomen, insbesondere 1 bis 10 Kohlenstoffatomen und vorzugsweise 1 bis 6 Kohlenstoffatomen, die gegebenenfalls mit einer oder mehreren Hydroxygruppen substituiert ist; ein Halogenatom, eine Carboxygruppe, eine Sulfogruppe;
R₃ und R₄ und/oder R₅ und R₆ können paarweise mit den Kohlenstoffatomen, an die sie gebunden sind, einen 6- bis 30-gliedrigen, aliphatischen oder aromatischen Cyclus oder Heterocyclus bilden, der gegebenenfalls mit einem identischen oder verschiedenen aromatischen Heterocyclus oder Cyclus mit 6 bis 30 Bestandteilen kondensiert ist; wobei die aliphatischen oder aromatischen Cyclen oder Heterocyclen gegebenenfalls mit mindestens einem Halogenatom; mit mindestens einer C₁₋₆-Alkoxygruppe, mit mindestens einer Carboxygruppe, mit mindestens einer Sulfogruppe und/oder mit mindestens einer C₆₋₃₀-Arylgruppe, mit mindestens einer geradkettigen oder verzweigten C₁₋₆-Alkylgruppe substituiert sind, wobei die Alkylgruppe gegebenenfalls durch eine Aminocarbonylgruppe oder eine Carbonylaminogruppe unterbrochen ist und gegebenenfalls von einer Hydroxygruppe, Carboxygruppe, Aminogruppe, Hydrogenocarbonylaminogruppe abgeschlossen wird;
B und B' bedeuten unabhängig voneinander ein Stickstoffatom oder die Gruppe CH;
W bedeutet eine zweiwertige Gruppe, die zwei Kohlenstoffatome derart enthält, dass die Verknüpfung N-W-B eine ungesättigte Bindung enthält oder nicht, wobei die zweiwertige Gruppe gegebenenfalls mit einer C₁₋₆-Alkylkette, Aryl(C₆₋₃₀)oxyalkyl(C₁₋₆) oder Alkyl(C₁₋₄)aryl(C₆₋₃₀)amino substituiert ist;
X und X' bedeuten unabhängig voneinander O, S, N, NR'₇, Se, CR'₈R'₉;
Y bedeutet O, S, N, Se, NR₇, CO, CR₈R₉;
R₈ und R₉ bedeuten unabhängig voneinander ein Wasserstoffatom; eine geradkettige oder verzweigte C₁₋₂₂-Alkylgruppe, die gegebenenfalls substituiert ist mit einer oder mehreren Gruppen Hydroxy, Mono- oder Dialkylamino mit C₁-C₁₀, Mono- oder Dihydroxyalkylamino mit C₁-C₁₀, Aryl(C₁₀₋₃₀), Aryloxy(C₁₀₋₃₀), Acyl(C₂₋₁₀)amino;
R₇ ist ein Wasserstoffatom, eine geradkettige oder verzweigte C₁₋₁₀-Alkylgruppe, eine C₆₋₃₀-Arylgruppe, eine Aminogruppe, die mindestens eine C₆₋₃₀-Arylgruppe, die gegebenenfalls mit mindestens einer C₁₋₆-Alkylgruppe oder mindestens einer Carboxygruppe substituiert ist, mindestens eine Alkyl(C₁₋₄)aryl(C₆₋₃₀)sulfonylgruppe oder mindestens eine C₂₋₁₀-Acylgruppe oder mindestens eine Trialkyl(C₁₋₄)-aminoalkyl(C₁₋₄)carbonylgruppe oder mindestens eine Aminothiocarbonylgruppe trägt;
R₇ kann auch eine Gruppe Z oder -N=Z bedeuten, wobei Z ein 5-oder 6-gliedriger Heterocyclus ist, der gegebenenfalls kondensiert ist und 1 bis 30 Kohlenstoffatome aufweist, wobei ein oder mehrere Kohlenstoffatome durch eine Gruppe CO ersetzt sein können;
R'₇ bedeutet ein Wasserstoffatom, eine C₁₋₆-Alkylgruppe, eine C₁₋₆-Hydroxyalkylgruppe;
R'₈ und R'₉ bedeuten unabhängig voneinander ein Wasserstoffatom, eine C₁₋₆-Alkylgruppe, eine C₁₋₆-Hydroxyalkylgruppe, eine C₁₋₆-Carboxyalkylgruppe, eine Alkoxy(C₁₋₄)carbonylalkyl(C₁₋₆)gruppe;
p bedeutet eine ganze Zahl 0 oder 1;
A- ist ein organisches oder anorganisches Anion oder ein Gemisch von Anionen.

2. Zusammensetzung nach dem vorhergehenden Anspruch, **dadurch gekennzeichnet, dass** die Gruppe R₁ ein Wasserstoffatom oder eine geradkettige oder verzweigte, unsubstituierte C₁₋₁₆-Alkylgruppe ist.

3. Zusammensetzung nach einem der vorhergehenden Ansprüche, **dadurch gekennzeichnet, dass** Y O, N, NR₇, CO bedeutet.

4. Zusammensetzung nach einem der vorhergehenden Ansprüche, **dadurch gekennzeichnet, dass** X und X' unabhängig voneinander ein Schwefelatom, ein Sauerstoffatom, eine Gruppe NR'₇ bedeuten, wobei R eine C₁₋₄-Alkylgruppe ist.

5. Zusammensetzung nach einem der vorhergehenden Ansprüche, **dadurch gekennzeichnet, dass** X und X' identisch sind.

6. Zusammensetzung nach einem der vorhergehenden Ansprüche, **dadurch gekennzeichnet, dass** W die Ethylengruppe bedeutet.

7. Zusammensetzung nach einem der vorhergehenden Ansprüche, **dadurch gekennzeichnet, dass** B und B' ein Kohlenstoffatom oder die Gruppe CH bedeuten.

8. Zusammensetzung nach einem der vorhergehenden Ansprüche, **dadurch gekennzeichnet, dass** R₃ und R₄ und/oder R₅ und R₆ paarweise mit den Kohlenstoffatomen, an die sie gebunden sind, einen aliphatischen oder aromatischen Cyclus oder Heterocyclus mit 6 bis 30 Bestandteilen bilden, welcher mit einem identischen oder nicht identischen aromatischen Cyclus oder Heterocyclus mit 6 bis 30 Bestandteilen kondensiert ist; wobei die aliphatischen oder aromatischen Cyclen oder Heterocyclen gegebenenfalls mit mindestens einem Halogenatom; mit mindestens einer C₁₋₆-Alkoxygruppe, mit mindestens einer Carboxygruppe, mit mindestens einer Sulfogruppe und/oder mit mindestens einer C₆₋₃₀-Arylgruppe, mit mindestens einer geradkettigen oder verzweigten C₁₋₆-Alkylgruppe substituiert sind, wobei die Alkylgruppe gegebenenfalls durch eine Aminocarbonyl- oder Carbonylaminogruppe unterbrochen und gegebenenfalls von einer Hydroxygruppe, Carboxygruppe, Aminogruppe oder Hydrogenocarbonylaminogruppe abgeschlossen wird.

9. Zusammensetzung nach einem der vorhergehenden Ansprüche, **dadurch gekennzeichnet, dass** das Anion anorganischer Herkunft unter den Halogeniden, Sulfaten, Bisulfaten, Nitraten, Phosphaten, Hydrogenphosphaten, Dihydrogenphosphaten, Carbonaten, Bicarbonaten ausgewählt ist; und **dadurch**, dass das Anion organischer Herkunft unter den Anionen ausgewählt ist, die von Salzen von Mono- oder Polycarbonsäuren, Sulfonsäuren, Schwefelsäuren, die gesättigt oder ungesättigt, aromatisch oder nicht aromatisch, gegebenenfalls mit mindestens einer Hydroxygruppe, Aminogruppe oder Halogenatomen substituiert sind, abgeleitet sind.

10. Zusammensetzung nach dem vorhergehenden Anspruch, **dadurch gekennzeichnet, dass** das Anion unter Chlorid, Iodid, Sulfat, Methosulfat, Ethosulfat ausgewählt ist.

11. Zusammensetzung nach einem der vorhergehenden Ansprüche, **dadurch gekennzeichnet, dass** der Direktfarbstoff einer der folgenden Verbindungen entspricht:
· 2,3,13,14-Tetrachlor-16,18-diethyl-6,7,7a,8,8a,9,10,16-octahydro-8-[(4-methylphenyl)amino]-benzimidazo[2,1-f]benzimidazo[1',2':1,2]pyrido[4;3-b] [1,6]naphthyridiniumiodid
· 8-(2-Benzothiazolylamino)-7,7a,8,8a,9,10-hexahydro-6H-benzothiazolo[2,3-f]benzothiazolo[3',2':1,2]pyrido[4,3-b] [1,6]naphthyridin-5-iumiodid
· 7,7a,8,8a,9,10-Hexahydro-8-[[(trimethylammonio)acetyl]amino]-6H-benzothiazolo[2,3-fJbenzothiazolo[3',2':1,2]pyrido[4,3-b][1,6]-naphthyridin-5-iumchloridiodid
· 7,7a,8,8a,8,9,10-Hexahydro-8-(4-oxo-2-thioxo-3-thiazolidinyl)-6H-benzothiazolo12 ,3-flbenzothiazolo[3',2':1,2]pyrido[4,3-b]-[1,6]naphthyridin-5-iumiodid
· 7,7a,8,8a,8,9,10-Hexahydro-8-[[(4-methylphenyl)sulfonyl]amino]-6H-benzothiazolo[2,3-f]benzothiazolo[3',2':1,2]pyrido[4,3-b]-1,6]naphthyridin- 5-iumiodid
· 8-[(1,1-Dioxido-1,2-benzisothiazol-3-yl)amino]-7,7a,8,8a,9,10-hexahydro-6H-benzothiazolo[2,3-fJbenzothiazolo[3',2':1,2]-pyrido[4,3-b] [1,6]naphthyridin-5-iumiodid
· 2,3,13,14-Tetrachlor-16,18-diethyl-7,7a,8,8a,9,10,16-hexahydro-6H-pyrano[3",2":3,4;5",6":3',4']dipyrido1,2-a:1',2'-a']bisbenz-imidazolium-trifluoracetat
· 17-Ethyl-7,7a,8a,9-tetrahydro-3,13-diphenyl-6H, 10H-pyrano[3",2":3,4;5",6":3',4']dipyrido[2,1-b:2',1'-b]bisbenzoxazol-5-iumbromid
· 7,7a,8a,9-Tetrahydro-3,13-dimethoxy-6H, 10H-pyrano-[3",2":3,4;5",6":3',4']dipyrido[2,1-b:2',1'-b]bisbenzothiazol-5-iumiodid
· 17-Ethyl-7,7a,8a,9-tetrahydro-6H- 10H-pyrano-[3",2":3,4;5",6":3',4']dipyrido[2,1-b:2',1'-b]bisbenzothiazol-5-iumbromid
· 7,7a,8a,9-Tetrahydro-6H,10H-pyrano[3",2":3,4;5",6":3',4']-dipyrido[2, 1-b:2', 1'-b]bisbenzothiazol-5-iumiodid
· 8-(2-Benzothiazolylamino)-7,7a,8,8a,9, 10-hexahydro-3,13-diphenyl-6H-benzoxazolo[2,3-f]benzoxazolo[3',2':1,2]pyrido[4,3-b] [1,6]naphthyridin-5-iumbromid
· 8-(2-Benzothiazolylamino)-7,7a,8,8a,9,10-hexahydro-6H-benzothiazolo[2,3-f]benzothiazolo[3',2':1,2]pyrido[4,3-b] [1,6]naphthyridin-5-iumbromid
· 8-[(Aminothioxomethyl)amino]-7,7a,8,8a,9,10-hexahydro-6H-benzothiazolo[2,3-f]benzothiazolo[3',2':1,2]pyrido[4,3-b][1,6]-naphthyridin-5-iumiodid
· 2,3,13,14-Tetrachlor-16,18-diethyl-7,7a,8,8a,9,10,16,18-octahydro-9-[(4-methylphenyl)amino]-6H-benzimidazo[2,1-f]benz-imidazo[1',2':1,2]pyrido[4,3-b][1,6]naphthyridin-5-iumiodid
· 6,7,11,12,18,20-Hexahydro-9-oxo-9H-bisbenzimidazo[2',1':3,4]-pyrazino[1,2-c:2',1'-f]pyrimidin-5-iumchlorid
· Inneres Salz von 2-(Aminomethyl)-6,7,9,10,16,18-hexahydro-16,16,18,18-tetramethyl-14-sulfo-pyrano(3",2":3,4;5",6":3',4']-dipyrido[1,2-a:1',2'-a']diindol-5-ium
· Inneres Salz von 2-(Carboxymethyl)-6,7,9,10,16,18-hexahydro-16,16,18,18-tetramethyl-14-sulfopyrano[3",2":3,4;5",6":3',4']-dipyrido[1,2-a:1',2'-a']diindol-5-ium
· Inneres Salz von 2,14-Bis(carboxymethyl)-6,7,9,10,16,18-hexahydro-16,16,18,18-tetramethyl-pyrano-[3",2":3,4;5",6":3',4']dipyrido[1,2-a:1',2'-a']diindol-5-ium
· Inneres Salz von 14-(Carboxymethyl)7,7a,8a,9,10,16-hexahydro-16,16-dimethyl-6H-benz[2", 3"]indolizino[8',7':5,6]pyrano[3,2-a]benzo[f]chinolizin-5-ium
· Inneres Salz von 8-[(4-Carboxyphenyl)amino]-7,7a,8,8a,9,10,16,18-octahydro-16,16,18,18-tetramethyl-6H-benz([2,3]indolizino[7,8-b]indolo[2,1-f][1,6]naphthyridin-5-ium
· Inneres Salz von 2-(Carboxymethyl)-6,7,7a,8a,9,18-hexahydro-18,18-dimethyl-10H-benz[2",3"]indolizino[8",7":5',6']pyrano-[3',2':3,4]pyrido[2,1-b]benzothiazol-5-ium
· Inneres Salz von 6,7,9,10,16,18-Hexahydro-2-(2-hydroxyethyl)-16,16,18,18-tetramethyl-14-sulfo-pyrano[3", 2":3,4;5",6":3',4']-dipyrido[I,2-a: 1 ',2'-a']diindol-5-ium
· Inneres Salz von 2-[(Formylamino)methyl]-6,7,9,10,16,18-hexahydro-16,16,18,18-tetramethyl- 14-sulfo-pyrano-[3",2":3,4;5",6":3',4']dipyrido[1,2-a:1',2'-a']diindol-5-ium
· Inneres Salz von 2-[2-[(2-Aminoethyl)amino]-2-oxoethyl]-6,7,9,10,16,18-hexahydro-16,16,18,18-tetramethyl-14-sulfopyrano[3",2":3,4;5",6":3',4']dipyrido[1,2-a:1',2'-a']diindol-5-ium
· Inneres Salz von 2-[2-[(2-Carboxymethyl)amino]-2-oxoethyl-6,7,9,10,16,18-hexahydro-16,16,18,18-tetramethyl-14-sulfopyrano[3",2":3,4;5",6":3',4']dipyrido[1,2-a:1',2'-a']diindol-5-ium
· Inneres Salz von 8,9,11,12,20,22-Hexahydro-20,20,22,22-tetramethyl-3,17-disulfo-dibenzo[e,e']pyrano-[3",2":3,4;5",6":3',4']dipyrido[1,2-a:1',2'-a']diindol-7-ium
· Salz (beispielsweise Chlorid) von 7H,11 H-Bisnaphth-[2',3':4,5]oxazolo[3,2-a:3',2'-a']pyrano[3,2-c:5,6-c']dipyridin-6-ium
· 20-Ethyl-8,8a,9a, 10-tetrahydro-8, 10-bis(3-phenoxypropyl)-7H, 11H-bisnaphth[2',3':4,5]oxazolo[3,2-a:3',2'-a']pyrano-[3,2-c:5,6-c']-dipyridin-6-ium-4-methylbenzolsulfonat
· Salz (beispielsweise das Chlorid) von 8,8a,9a,10-Tetrahydro-8,10-bis(3-phenoxypropyl)-7H, 11H-bisnaphth[2',3':4,5]oxazolo-[3,2-a:3',2'-a']pyrano[3,2-c:5,6-c']dipyridin-6-ium
· Salz (beispielsweise Chlorid) von 6H, 10H-Naphtho[1"",2"":4"',5"']-thiazolo[3"',2"',1",2"]pyrido[3",4":5',6']pyrano[3',2':3,4]pyrido[2,1-b]benzoxazol-5-
· 7,7a,8a,9-Tetrahydro-6H-10H-pyrano[3",2":3,4;5",6":3',4']-dipyrido[2,1-b:2',1'-b']bisbenzothiazol-5-iumbromid
· Salz (beispielsweise Chlorid) von 10H-Benz([2",3"]indolizino-[8",7":5',6']pyrano[3',2':3,4]pyrido[2,1-b]benzothiazol-5-ium
· Salz (beispielsweise Chlorid) von Dibenzo[e,e']pyrano-[3",2":3,4;5",6":3',4']dipyrido[1,2-a:1',2'-a']diindol-7
· Salz (beispielsweise Chlorid) von Pyrano[3",2":3,4;5",6":3',4']-dipyrido[1,2-a:1',2'-a']diindol-5-ium
· Salz (beispielsweise Chlorid) von 1H-Benz[2,3]indolizino[7,8-b]indolo[2,1-f][1,6]naphthyridin-5-ium
· 7,7a,8a,9-Tetrahydro-6H, 10H-pyrano[3",2":3,4;5",6":3',4']-dipyrido[2,1-b:2', 1'-b']bisbenzothiazol-5-ium-heptafluorbutanoat
· 2,3,13,14-Tetrachlor-16,18-diethyl-7,7a,8a,9,10,16-hexahydro-6H-pyranol[3",2":3,4;5",6":3',4']dipyrido[1,2-a:1',2'-a']bis-benzimidazoliumsulfat
· Salz (beispielsweise Chlorid) von 8-(Acetylamino)-7,7a,8,8a,9,10-hexahydro-6H-benzothiazolo[2,3-f]benzothiazolo[3',2':1,2]-pyrido[4,3-b] [1,6]naphthyridin-5-ium
· Salz (beispielsweise Chlorid) von 1H-Benzimidazo[2,1-f]-benzirnidazo[1',2':1,2]pyrido[4,3-b][1,6]naphthyridin-5-ium
· 2,3,13,14-Tetrachlor-16,18-diethyl-6,7,7a,8,8a,9,10,16-octahydro-8-[(4-methylphenyl)amino]-benzimidazo[2,1-f]benzimidazo[1',2':1,2]pyrido[4,3-b][1,6]naphthyridiniumiodid
· Salz (beispielsweise Chlorid) von Pyrano[3",2":3,4;5",6",3',4']-dipyrido[1,2-a:1',2'-a']bisbenzimidazol-5-
· Salz (beispielsweise Chlorid) von 6H,10H-Pyrano-[3",3":3,4;5",6",3',4']dipyrido[2,1-b:2',1'-b']bisbenzothiazol-5-ium
· Salz (beispielsweise Chlorid) von 6H-Benzothiazolo[2,3-f]-benzothiazolo[3',2':1,2]pyrido[4,3-b][1,6]naphthyridin-5-ium
· Salz (beispielsweise Chlorid) von 6H,10H-Pyrano-[3",2":3,4;5",6":3',4']dipyrido[2,1-b:2',1'-b']bisbenzoxazolo-5-
· Salz (beispielsweise Chlorid) von 6H-Benzoxazolo[2,3-f]-benzoxazolo[3',2':1,2]pyrido[4,3-b][1,6]naphthyridin-5-ium
· Salz (beispielsweise Chlorid) von 6H-Benzimidazo[2,1-f]-benzimidazo[1',2':1,2]pyrido[4,3-b][1,6]naphthyridin-5-ium
· 8-(Acetylamino)-7,7a,8,8a,9,10-hexahydro-6H-benzothiazolo[2,3-f]benzothiazolo[3',2':1,2]pyrido[4,3-b][1,6]naphthyridin-5-iumdichloriodat
· 6,7,11,12-Tetrahydro-9-oxo-9H-bisthiazolo[3,2-a:3',2'-a']-pyrimido[6,1-c:4,3-c']dipyrazin-5-iumchlorid
· 6,7,11,12-Tetrahydro-9-oxo-9H-bisoxazolo[3,2-a:3',2'-a']-pyrimido[6,1-c:4,3-c']dipyrazin-5-iumbromid
· 6,7,11,12-Tetrahydro-9-oxo-9H-bisselenazolo[3,2-a;3',2'-a']-pyrimido[6,1-c:4,3-c']dipyrazin-5-iumbromid.

12. Zusammensetzung nach einem der vorhergehenden Ansprüche, **dadurch gekennzeichnet, dass** der Mengenanteil an Direktfarbstoff(en) der Formeln (I) oder (I') im Bereich von 0,01 bis 20 Gew.-%, bezogen auf das Gesamtgewicht der Zusammensetzung, liegt.

13. Zusammensetzung nach dem vorhergehenden Anspruch, **dadurch gekennzeichnet, dass** der Mengenanteil an Direktfarbstoff(en) der Formeln (I) oder (I') im Bereich von 0,1 bis 5 Gew.-%, bezogen auf das Gesamtgewicht der Zusammensetzung, liegt.

14. Zusammensetzung nach einem der vorhergehenden Ansprüche, **dadurch gekennzeichnet, dass** sie ferner mindestens einen ergänzenden Direktfarbstoff nichtionischer, kationischer oder anionischer Natur enthält.

15. Zusammensetzung nach dem vorhergehenden Anspruch, **dadurch gekennzeichnet, dass** die ergänzenden Direktfarbstoffe unter den nitrierten Benzolfarbstoffen, Azofarbstoffen, AnthrachinonFarbstoffen, Naphthochinon-Farbstoffen oder Benzochinon-Farbstoffen, Indigoiden oder von Triarylmethan abgeleiteten Farbstoffen, natürlichen Farbstoffen oder deren Gemischen ausgewählt sind.

16. Zusammensetzung nach einem der Ansprüche 14 oder 15, **dadurch gekennzeichnet, dass** der oder die ergänzenden Direktfarbstoffe 0,0005 bis 12 Gew.-%, bezogen auf das Gesamtgewicht der Zusammensetzung, ausmachen.

17. Zusammensetzung nach Anspruch 1, **dadurch gekennzeichnet, dass** der grenzflächenaktive Stoff nichtionisch ist.

18. Zusammensetzung nach einem der vorhergehenden Ansprüche, **dadurch gekennzeichnet, dass** der Mengenanteil der grenzflächenaktiven Stoffe 0,01 bis 50 Gew.-%, bezogen auf das Gesamtgewicht der Zusammensetzung, ausmacht.

19. Zusammensetzung nach einem der vorhergehenden Ansprüche, **dadurch gekennzeichnet, dass** sie mindestens ein nichtassoziatives verdickendes Polymer enthält.

20. Zusammensetzung nach dem vorhergehenden Anspruch, **dadurch gekennzeichnet, dass** sie mindestens ein nichtassoziatives verdickendes Polymer enthält, das, einzeln oder in Form von Gemischen, unter den vernetzten Homopolymeren von Acrylsäure, vernetzten Homopolymeren von 2-Acrylamido-2-methyl-propansulfonsäure und ihren vernetzten Copolymeren mit Acrylamid, Homopolymeren von Ammoniumacrylat oder Copolymeren von Ammoniumacrylat und Acrylamid, nichtionischen Guargummen, Biopolysacchariden mikrobieller Herkunft, aus Pflanzenexsudaten stammenden Gummen, Hydroxypropyl- oder Carboxymethylcellulose; Pektinen und Alginaten ausgewählt ist.

21. Zusammensetzung nach einem der vorhergehenden Ansprüche, **dadurch gekennzeichnet, dass** sie mindestens ein assoziatives verdickendes Polymer enthält.

22. Zusammensetzung nach dem vorhergehenden Anspruch, **dadurch gekennzeichnet, dass** sie mindestens ein assoziatives verdickendes Polymer enthält, das, einzeln oder in Form von Gemischen, unter den assoziativen Polyurethanen, die insbesondere kationisch oder nichtionisch sind, assoziativen Cellulosederivaten, die insbesondere kationisch oder nichtionisch sind, assoziativen Vinyllactamen, assoziativen ungesättigten Polysäuren, assoziativen Aminoplastethern, vernetzten Homopolymeren von 2-Acrylamido-2-methylpropansulfonsäure und ihren vernetzten Copolymeren mit Acrylamid, assoziativen Polymeren oder Copolymeren, die mindestens ein Monomer mit ethylenisch ungesättigter Bindung und Sulfonsäuregruppe enthalten, ausgewählt ist.

23. Zusammensetzung nach einem der Ansprüche 19 bis 22, **dadurch gekennzeichnet, dass** der Mengenanteil des assoziativen oder nichtassoziativen verdickenden Polymers 0,01 bis 10 Gew.-% und insbesondere 0,1 bis 5 Gew.-%, bezogen auf das Gewicht der Zusammensetzung, ausmacht.

24. Zusammensetzung nach einem der vorhergehenden Ansprüche, **dadurch gekennzeichnet, dass** sie in Form eines färbenden Haarwaschmittels vorliegt.

25. Zusammensetzung nach einem der Ansprüche 1 bis 23, **dadurch gekennzeichnet, dass** sie mindestens eine Oxidationsbase gegebenenfalls in Kombination mit mindestens einem Kuppler enthält.

26. Zusammensetzung nach dem Anspruch 25, **dadurch gekennzeichnet, dass** sie mindestens eine Oxidationsbase enthält, die unter den para-Phenylendiaminen, Bisphenylalkylendiaminen, para-Aminophenolen, ortho-Aminophenolen und heterocyclischen Basen und ihren Additionssalzen mit einer Säure oder mit einem alkalischen Stoff ausgewählt ist.

27. Zusammensetzung nach dem vorhergehenden Anspruch, **dadurch gekennzeichnet, dass** der Mengenanteil der Oxidationsbase(n) 0,0005 bis 12 Gew.-%, bezogen auf das Gesamtgewicht der Zusammensetzung, ausmacht.

28. Zusammensetzung nach einem der Ansprüche 25 bis 27, **dadurch gekennzeichnet, dass** sie mindestens einen Kuppler enthält, der unter den meta-Phenylendiaminen, meta-Aminophenolen, meta-Dihydroxybenzolen und heterocyclischen Kupplern oder ihren Additionssalzen mit einer Säure oder mit einem alkalischen Stoff ausgewählt ist.

29. Zusammensetzung nach dem vorhergehenden Anspruch, **dadurch gekennzeichnet, dass** der Mengenanteil des Kupplers oder der Kuppler 0,0001 bis 10 Gew.-%, bezogen auf das Gesamtgewicht der Farbmittelzusammensetzung, ausmacht.

30. Zusammensetzung nach einem der vorhergehenden Ansprüche, **dadurch gekennzeichnet, dass** sie mindestens einen Zusatzstoff enthält, der unter den anionischen, kationischen, nichtionischen, amphoteren, zwitterionischen Polymeren oder deren Gemischen; anorganischen Verdickungsmitteln; Antioxidantien; Penetrationsmitteln; Maskierungsmitteln; Parfums; Puffern; Dispergiermitteln; Konditioniermitteln; Filmbildnern; Ceramiden; Konservierungsmitteln; Stabilisatoren; Trübungsmitteln ausgewählt ist.

31. Zusammensetzung nach einem der Ansprüche 1 bis 23 und 25 bis 30, **dadurch gekennzeichnet, dass** sie mindestens ein Oxidationsmittel enthält.

32. Zusammensetzung nach dem vorhergehenden Anspruch, **dadurch gekennzeichnet, dass** das Oxidationsmittel unter Wasserstoffperoxid, Harnstoffperoxid, Alkalimetallbromaten, Persalzen, wie Perboraten und Persulfaten, und Enzymen ausgewählt ist.

33. Verfahren zur Behandlung von Keratinfasern und insbesondere menschlichen Keratinfasern, **dadurch gekennzeichnet, dass** eine Zusammensetzung, wie sie in einem der vorhergehenden Ansprüche definiert ist, während einer Zeitspanne, die für die Bildung der Färbung ausreichend ist, auf die trockenen oder feuchten Fasern aufgebracht wird, worauf gespült, gegebenenfalls mit Haarwaschmittel gewaschen, nochmals gespült und die resultierenden Fasern getrocknet oder trocknen gelassen werden.

34. Verfahren für die Behandlung von Keratinfasern und insbesondere menschlichen Keratinfasern, **dadurch gekennzeichnet, dass** eine Zusammensetzung, wie sie in einem der Ansprüche 1 bis 23 definiert ist, ohne dass an Ende gespült wird, auf die trockenen oder feuchten Fasern aufgebracht wird.

35. Vorrichtung mit mehreren Abteilungen zum Färben und Aufhellen von Haaren, die mindestens eine Abteilung mit einer Zusammensetzung nach einem der Ansprüche 1 bis 23 und 25 bis 30 und mindestens eine weitere Abteilung aufweist, die eine Zusammensetzung mit mindestens einem Oxidationsmittel enthält.

36. Verwendung der Zusammensetzung nach einem der Ansprüche 1 bis 32 als Farbmittel für Keratinfasern und insbesondere menschliche Keratinfasern.
